# EUROPEAN PATENT APPLICATION

(11) **EP 4 548 780 A1**
(43) Date of publication of application: **07.05.2025**
(21) Application number: 23811906.9
(22) Date of filing: 26.05.2023
(51) Int. Cl.: A23L 33/135, A61K 35/744, A61K 35/747, A61P 17/16, A61P 25/28, A61P 29/00, A61P 37/02

(54) **FPR2 AGONIST CONTAINING OUTER MEMBRANE VESICLES DERIVED FROM LACTIC ACID BACTERIUM**

(30) Priority: 27.05.2022 JP 2022086832
(71) Applicant: Asahi Group Holdings, Ltd., Tokyo 130-8602 (JP)
(72) Inventor: SUGAHARA, Hirosuke, Moriya-shi, Ibaraki 302-0106 (JP); NAGAYAMA, Keitaro, Moriya-shi, Ibaraki 302-0106 (JP)
(74) Representative: J A Kemp LLP
(86) International application number: PCT/JP2023/019763
(87) International publication number: WO 2023/229034

(57) **Abstract**

The present invention provides an FPR2 agonist. Specifically, the FPR2 agonist comprises, as an active ingredient, outer membrane vesicles derived from at least one lactic acid bacterium selected from the group consisting of genus Lactobacillus, genus Lacticaseibacillus, genus Lactiplantibacillus, genus Ligilactobacillus, genus Lactococcus, and Streptococcus salivarius subsp. thermophilus.

## Description

### Technical Field

The present invention relates to an invention characterized in that outer membrane vesicles derived from lactic acid bacteria are used to activate FPR2.

### Background Art

Outer membrane vesicles are substances covered with a lipid bilayer membrane that are released from various cells. Outer membrane vesicles can pass through the blood-brain barrier (Non Patent Literatures 5 and 6). Outer membrane vesicles are stable even after being taken up by host cells (Non Patent Literature 7). Outer membrane vesicles having these characteristics are expected to be applied to drug delivery systems (Non Patent Literatures 6 and 7).

Bacteria also release outer membrane vesicles. Outer membrane vesicles derived from lactic acid bacteria have physiological activities, such as suppressing the secretion of inflammatory mediators (IL-6, etc.), inducing differentiation into M2 macrophages, suppressing inflammatory responses by macrophages, and enhancing the host immune response to vancomycin-resistant enterococci (Patent Literatures 1 to 6 and Non Patent Literatures 1 and 2).

It has also been reported that outer membrane vesicles derived from Lactiplantibacillus plantarum induce IL-10 (Non Patent Literature 14) and that outer membrane vesicles derived from Lacticaseibacillus paracasei (former classification name: Lactobacillus paracasei) alleviate inflammatory responses in the intestine (Non Patent Literature 15).

Formyl Peptide Receptor (FPR) is a receptor for formylmethionyl peptides known as chemotactic factors.

There are three FPR families (FPR1, FPR2, and FPR3) in humans. Although there are various ligands for FPR, the selectivity for each FPR differs depending on the ligand (Non Patent Literature 3).

Various substances are known to act as FPR2 agonists or antagonists (Non Patent Literature 3).

FPR2 regulates both innate immunity mediated by phagocytes and acquired immunity mediated by B cells and the like (Patent Literature 7).

FPR2 plays an important role in host defense and inflammatory responses and is recognized as a target for therapeutic agents for inflammatory diseases and the like (Non Patent Literature 4).

FPR2 agonists are known as therapeutic agents for diseases associated with FPR2 (such as inflammatory diseases) (Patent Literature 7).

FPR2 (FPRL-1) is also a functional receptor for amyloid β42 (Aβ42), which is important in the pathophysiology of neurotoxicity associated with Alzheimer's disease (Non Patent Literature 8). It has been suggested that the peptide Humanin (HN), which binds to FPR2, can exert a neuroprotective effect by competitively inhibiting access of FPR2 to Aβ42 (Non Patent Literature 8). Humanin (HN) acts more strongly on FPR2 than on FPR1 (Non Patent Literature 9).

FPR2 is expressed in epithelial cells of the colon and mediates N-formyl peptidedependent epithelial cell proliferation and regeneration. In addition, FPR2 plays an important role in maintaining homeostasis, inflammation, and mediating epithelial repair in the colon (Non Patent Literature 10).

The usefulness of FPR2 in the development of therapeutic agents for chronic inflammatory bowel disease has been suggested because a ligand of FPR2 (FPRL1) has a therapeutic effect on ulcerative colitis (Non Patent Literature 11).

FPR2 agonists have also been demonstrated to be beneficial in preclinical models of chronic inflammatory human diseases, including infectious diseases, psoriasis, dermatitis, inflammatory bowel syndrome, Crohn's disease, ocular inflammation, sepsis, pain, metabolism/diabetes, cancer, COPD, asthma and allergic diseases, cystic fibrosis, acute lung injury and fibrosis, rheumatoid arthritis and other joint diseases, Alzheimer's disease, renal fibrosis, and organ transplantation (Patent Literature 7 and Non Patent Literatures 12 and 13).

### Citation List

### Patent Literatures

Patent Literature 1: International Application Japanese-Phase Publication No. 2021-516054
Patent Literature 2: International Application Japanese-Phase Publication No. 2021-513336
Patent Literature 3: International Application Japanese-Phase Publication No. 2021-502970
Patent Literature 4: International Application Japanese-Phase Publication No. 2021-501136
Patent Literature 5: International Application Japanese-Phase Publication No. 2018-511583
Patent Literature 6: International Application Japanese-Phase Publication No. 2013-530216
Patent Literature 7: International Application Japanese-Phase Publication No. 2021-516240

### Non Patent Literatures

Non Patent Literature 1: Uragami Foundation Research Report, Vol. 24, p. 46-52, 2017
Non Patent Literature 2: BMC Microbiology, 17:66, 2017
Non Patent Literature 3: Molecules, 22, 455, 2017
Non Patent Literature 4: NATURE COMMUNICATIONS, 11:1208, 2020
Non Patent Literature 5: Int. J. Mol. Sci., 21, 4407, 2020
Non Patent Literature 6: Cell Biosci., 11:142, 2021
Non Patent Literature 7: KAGAKU TO SEIBUTSU, Vol. 54, No. 11, p. 812-819, 2016
Non Patent Literature 8: Front. Endocrinol., Volume 5, Article 210, December 2014
Non Patent Literature 9: Biochemical and Biophysical Research Communications 324 (2004) 255-261
Non Patent Literature 10: J Clin Invest. 2013;123(4):1694-1704
Non Patent Literature 11: Experimental & Molecular Medicine (2013) 45, e40
Non Patent Literature 12: Eur. J. Pharmacol., 2015, 5, 49-63
Non Patent Literature 13: Trends in Pharm. Sci., 2015, 36, 737-755
Non Patent Literature 14: Scientific Reports, Vol. 12, Article number: 13330 (2022)
Non Patent Literature 15: Experimental & Molecular Medicine, 52: 423-437 (2020)

### Summary of Invention

### Problems to be solved by the invention

Substances that activate FPR2 are considered to be useful for the treatment of diseases associated with FPR2.

Outer membrane vesicles have properties (such as excellent stability) that are advantageous for the aforementioned treatment.

However, outer membrane vesicles that activate FPR2 are unknown.

Accordingly, an object of the present invention is to provide outer membrane vesicles capable of activating FPR2.

### Means for solution of the problems

As a result of intensive studies on this object, the present inventors have found that outer membrane vesicles derived from a specific species of lactic acid bacterium activate FPR2. The present invention has been made based on this finding.

That is, the present invention relates to the following [1] to [36].
[1] An FPR2 agonist comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus.
[2] The FPR2 agonist according to [1], wherein the lactic acid bacterium is at least one selected from the group consisting of the following:
   Lactobacillus delbrueckii subsp. bulgaricus,
   Lactobacillus helveticus,
   Lactobacillus acidophilus,
   Lacticaseibacillus rhamnosus,
   Lacticaseibacillus paracasei,
   Lactiplantibacillus plantarum,
   Ligilactobacillus salivarius,
   Lactococcus lactis subsp. lactis,
   Streptococcus salivarius subsp. thermophilus,
   Lactobacillus gasseri,
   Lactobacillus amylovorus,
   Lactobacillus crispatus,
   Lactobacillus johnsonii,
   Lacticaseibacillus casei, and
   Lactiplantibacillus pentosus.
[3] The FPR2 agonist according to [1] or [2], wherein the outer membrane vesicles derived from the lactic acid bacterium do not have FPR1 agonistic activity.
[4] A food and/or drink for FPR2 activation, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[5] A pharmaceutical composition for treating a disease associated with FPR2, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[6] A cosmetic composition for FPR2 activation, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[7] A consumer product composition for FPR2 activation, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[8] The food and/or drink according to [4], the pharmaceutical composition according to [5], the cosmetic composition according to [6], or the consumer product composition according to [7], wherein the outer membrane vesicles derived from the lactic acid bacterium do not have FPR1 agonistic activity.
[9] Lacticaseibacillus paracasei CP3526 strain having accession number NITE BP-03625.
[10] A food and/or drink for inducing IL-10 by FPR2 activation, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[11] A pharmaceutical composition for treating a disease associated with IL-10 by IL-10 induction by FPR2 activation, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[12] A cosmetic composition for inducing IL-10 by FPR2 activation, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[13] A consumer product composition for inducing IL-10 by FPR2 activation, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[14] The food and/or drink according to [10], the pharmaceutical composition according to [11], the cosmetic composition according to [12], or the consumer product composition according to [13], wherein the outer membrane vesicles derived from the lactic acid bacterium do not have FPR1 agonistic activity.
[15] A food and/or drink for protecting the skin from ultraviolet rays, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[16] A pharmaceutical composition for protecting the skin from ultraviolet rays, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[17] A cosmetic composition for protecting the skin from ultraviolet rays, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[18] A consumer product composition for protecting the skin from ultraviolet rays, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[19] The food and/or drink according to [15], the pharmaceutical composition according to [16], the cosmetic composition according to [17], or the consumer product composition according to [18], which protects the skin from ultraviolet rays by FPR2 activation.
[20] The food and/or drink according to [15], the pharmaceutical composition according to [16], the cosmetic composition according to [17], or the consumer product composition according to [18], wherein the outer membrane vesicles derived from the lactic acid bacterium do not have FPR1 agonistic activity.
[21] A food and/or drink for protecting neuronal cells from neurocytotoxicity, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[22] A pharmaceutical composition for protecting neuronal cells from neurocytotoxicity, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[23] A cosmetic composition for protecting neuronal cells from neurocytotoxicity, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[24] A consumer product composition for protecting neuronal cells from neurocytotoxicity, comprising outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus as an active ingredient.
[25] The food and/or drink according to [21], the pharmaceutical composition according to [22], the cosmetic composition according to [23], or the consumer product composition according to [24], which protects neuronal cells from neurocytotoxicity by FPR2 activation.
[26] The food and/or drink according to [21], the pharmaceutical composition according to [22], the cosmetic composition according to [23], or the consumer product composition according to [24], wherein the neurocytotoxicity is neurocytotoxicity caused by amyloid β.
[27] The food and/or drink according to [21], the pharmaceutical composition according to [22], the cosmetic composition according to [23], or the consumer product composition according to [24], wherein the outer membrane vesicles derived from the lactic acid bacterium do not have FPR1 agonistic activity.
[28] A method of activating FPR2 in a subject, comprising
   administering to the subject outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus.
[29] A method of treating a disease associated with FPR2 in a subject, comprising
   administering to the subject outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus.
[30] A method of inducing IL-10 by FPR2 activation in a subject, comprising
   administering to the subject outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus.
[31] A method of protecting the skin from ultraviolet rays in a subject, comprising
   administering to the subject outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus.
[32] The method according to [31], which protects the skin from ultraviolet rays by FPR2 activation.
[33] A method of protecting neuronal cells from neurocytotoxicity in a subject, comprising
   administering to the subject outer membrane vesicles derived from at least one lactic acid bacterium selected from the group consisting of
   genus Lactobacillus,
   genus Lacticaseibacillus,
   genus Lactiplantibacillus,
   genus Ligilactobacillus,
   genus Lactococcus, and
   Streptococcus salivarius subsp. thermophilus.
[34] The method according to [33], which protects neuronal cells from neurocytotoxicity by FPR2 activation.
[35] The method according to any one of [28] to [34], wherein the lactic acid bacterium is at least one selected from the group consisting of the following:
   Lactobacillus delbrueckii subsp. bulgaricus,
   Lactobacillus helveticus,
   Lactobacillus acidophilus,
   Lacticaseibacillus rhamnosus,
   Lacticaseibacillus paracasei,
   Lactiplantibacillus plantarum,
   Ligilactobacillus salivarius,
   Lactococcus lactis subsp. lactis,
   Streptococcus salivarius subsp. thermophilus,
   Lactobacillus gasseri,
   Lactobacillus amylovorus,
   Lactobacillus crispatus,
   Lactobacillus johnsonii,
   Lacticaseibacillus casei, and
   Lactiplantibacillus pentosus.
[36] The method according to any one of [28] to [35], wherein the outer membrane vesicles derived from the lactic acid bacterium do not have FPR1 agonistic activity.

### Advantageous Effects of Invention

As shown in Examples described below, outer membrane vesicles derived from specific lactic acid bacteria activate FPR2. Furthermore, as shown in Examples described below, outer membrane vesicles derived from specific lactic acid bacteria induce IL-10 via activation of FPR2, protect the skin from ultraviolet rays, and protect neuronal cells from neurocytotoxicity. The present invention using these outer membrane vesicles as an active ingredient is useful for the treatment of diseases associated with FPR2 and the like.

### Brief Description of Drawings

Fig. 1 shows FPR2 agonistic activity of outer membrane vesicles derived from 26 species of bacteria.
Fig. 2 shows FPR1 agonistic activity of outer membrane vesicles derived from 26 species of bacteria.
Fig. 3 shows the effect of known FPR2 agonists on IL-10 production by M2 macrophages.
Fig. 4 shows the IL-10 inducing activity of outer membrane vesicles derived from 17 species of bacteria.
Fig. 5 shows the effect of an FPR2 antagonist on the IL-10 inducing ability of outer membrane vesicles having FPR2 agonistic activity.
Fig. 6 shows the effect of outer membrane vesicles having FPR2 agonistic activity on protection of the skin from ultraviolet rays.
Fig. 7 shows the effect of outer membrane vesicles having FPR2 agonistic activity on protection of neuronal cells from neurocytotoxicity.

### Description of Embodiments

### [Lactic Acid Bacteria]

The outer membrane vesicles used in the present invention are derived from lactic acid bacteria (1) to (6) below.
(1) Genus Lactobacillus
(2) Genus Lacticaseibacillus
(3) Genus Lactiplantibacillus
(4) Genus Ligilactobacillus
(5) Genus Lactococcus
(6) Streptococcus salivarius subsp. thermophilus

The classification of lactic acid bacteria in the present specification follows the reclassification published in 2020. Details of the reclassification are described in Int J Syst Evol Microbiol. 2020 Apr; 70(4): 2782-2858, on the website of the Japan Bifidus Foundation (URL: https://bifidus-fund.jp/keyword/kw054.shtml), and the like.

Any known lactic acid bacterial strain that belongs to any one of (1) to (6) above and secretes outer membrane vesicles can be used in the present invention without particular limitation. Since outer membrane vesicles exert their effects after being taken or ingested, bacterial strains whose safety in animals has been confirmed are preferable.

Preferable lactic acid bacteria are lactic acid bacteria of (1) genus Lactobacillus, (2) genus Lacticaseibacillus, (3) genus Lactiplantibacillus, (4) genus Ligilactobacillus, or (5) genus Lactococcus.

More preferable lactic acid bacteria are lactic acid bacteria of (1) genus Lactobacillus, (2) genus Lacticaseibacillus, (3) genus Lactiplantibacillus, or (4) genus Ligilactobacillus.

Further preferable lactic acid bacteria are lactic acid bacteria of (1) genus Lactobacillus, (2) genus Lacticaseibacillus, or (4) genus Ligilactobacillus.

Particularly preferable lactic acid bacteria are lactic acid bacteria of (1) genus Lactobacillus or (2) genus Lacticaseibacillus.

### (1) Lactic Acid Bacteria of Genus Lactobacillus

Examples of lactic acid bacteria of the genus Lactobacillus include the following.
Lactobacillus delbrueckii subsp. bulgaricus
Lactobacillus helveticus
Lactobacillus acidophilus
Lactobacillus delbrueckii
Lactobacillus crispatus
Lactobacillus gasseri
Lactobacillus paragasseri
Lactobacillus amylovorus
Lactobacillus johnsonii

Preferable lactic acid bacteria of the genus Lactobacillus are Lactobacillus delbrueckii subsp. bulgaricus, Lactobacillus helveticus, Lactobacillus acidophilus, Lactobacillus delbrueckii, or Lactobacillus gasseri.

In one aspect of the present invention, preferable lactic acid bacteria of the genus Lactobacillus are Lactobacillus gasseri, Lactobacillus amylovorus, Lactobacillus crispatus, or Lactobacillus johnsonii.

In another aspect of the present invention, preferable lactic acid bacteria of the genus Lactobacillus are Lactobacillus delbrueckii subsp. bulgaricus, Lactobacillus helveticus, Lactobacillus acidophilus, Lactobacillus delbrueckii, Lactobacillus gasseri, Lactobacillus amylovorus, Lactobacillus crispatus, or Lactobacillus johnsonii.

Further preferable lactic acid bacteria of the genus Lactobacillus are Lactobacillus delbrueckii subsp. bulgaricus, Lactobacillus helveticus, or Lactobacillus acidophilus.

In one aspect of the present invention, further preferable lactic acid bacteria of the genus Lactobacillus are Lactobacillus delbrueckii subsp. bulgaricus, Lactobacillus helveticus, Lactobacillus acidophilus, Lactobacillus gasseri, Lactobacillus amylovorus, Lactobacillus crispatus, or Lactobacillus johnsonii.

Still further preferable lactic acid bacteria of the genus Lactobacillus are Lactobacillus delbrueckii subsp. bulgaricus, Lactobacillus helveticus, Lactobacillus acidophilus, Lactobacillus gasseri, or Lactobacillus johnsonii.

Lactobacillus delbrueckii subsp. bulgaricus may be any available bacterial strain and may include the type strain Lactobacillus delbrueckii subsp. bulgaricus T.

Lactobacillus helveticus may be any available bacterial strain and may include the type strain Lactobacillus helveticus T.

Lactobacillus acidophilus may be any available bacterial strain and may include the type strain Lactobacillus acidophilus T. Lactobacillus acidophilus may include other bacterial strains, such as strain JCM1023 and strain CP1613. Lactobacillus acidophilus strain CP1613 is a species of lactic acid bacterium belonging to Lactobacillus acidophilus, and has been internationally deposited with the NITE Patent Microorganisms Depositary of the National Institute of Technology and Evaluation (Room 122, 2-5-8 Kazusa-Kamatari, Kisarazu-shi, Chiba 292-0818, Japan), an international depositary authority under the Budapest Treaty, under accession number NITE BP-1513 on January 18, 2013.

Lactobacillus gasseri may be any available bacterial strain and may include the type strain Lactobacillus gasseri T. Lactobacillus gasseri may include other bacterial strains, such as strain CP2305. Lactobacillus gasseri strain CP2305 is a species of lactic acid bacterium belonging to Lactobacillus gasseri, and has been internationally deposited with the NITE Patent Microorganisms Depositary of the National Institute of Technology and Evaluation (Room 120, 2-5-8 Kazusa-Kamatari, Kisarazu-shi, Chiba 292-0818, Japan), an international depositary authority under the Budapest Treaty, under accession number FERM BP-11331 (deposited on September 11, 2007).

Lactobacillus amylovorus may be any available bacterial strain and may include the type strain Lactobacillus amylovorus T.

Lactobacillus crispatus may be any available bacterial strain and may include the type strain Lactobacillus crispatus T.

Lactobacillus johnsonii may be any available bacterial strain and may include the type strain Lactobacillus johnsonii T.

### (2) Lactic Acid Bacteria of Genus Lacticaseibacillus

Examples of lactic acid bacteria of the genus Lacticaseibacillus include the following.
Lacticaseibacillus rhamnosus
Lacticaseibacillus paracasei
Lacticaseibacillus casei
Lacticaseibacillus paracasei subsp. paracasei

Preferable lactic acid bacteria of the genus Lacticaseibacillus are Lacticaseibacillus rhamnosus, Lacticaseibacillus paracasei, or Lacticaseibacillus casei.

Further preferable lactic acid bacteria of the genus Lacticaseibacillus are Lacticaseibacillus paracasei or Lacticaseibacillus casei.

Lacticaseibacillus rhamnosus may be any available bacterial strain and may include the type strain Lacticaseibacillus rhamnosus T.

Lacticaseibacillus paracasei may be any available bacterial strain and may include the type strain Lacticaseibacillus paracasei T. Lacticaseibacillus paracasei may include other bacterial strains, such as strain JCM1133 and strain CP3526. Lacticaseibacillus paracasei strain CP3526 is a species of lactic acid bacterium belonging to Lacticaseibacillus paracasei, and has been internationally deposited with the NITE Patent Microorganisms Depositary of the National Institute of Technology and Evaluation (Room 122, 2-5-8 Kazusa-Kamatari, Kisarazu-shi, Chiba 292-0818, Japan), an international depositary authority under the Budapest Treaty, under accession number NITE BP-03625 on March 18, 2022.

Lacticaseibacillus casei may be any available bacterial strain and may include the type strain Lacticaseibacillus casei T.

In one aspect of the present invention, the lactic acid bacterium of the genus Lacticaseibacillus may be a lactic acid bacterium other than Lacticaseibacillus paracasei.

### (3) Lactic Acid Bacteria of Genus Lactiplantibacillus

Examples of lactic acid bacteria of the genus Lactiplantibacillus include the following.
Lactiplantibacillus plantarum
Lactiplantibacillus pentosus
Lactiplantibacillus paraplantarum

Preferable lactic acid bacteria of the genus Lactiplantibacillus are Lactiplantibacillus plantarum or Lactiplantibacillus pentosus.

Further preferable lactic acid bacteria of the genus Lactiplantibacillus are Lactiplantibacillus plantarum.

Lactiplantibacillus plantarum may be any available bacterial strain and may include the type strain Lactiplantibacillus plantarum T.

Lactiplantibacillus pentosus may be any available bacterial strain and may include the type strain Lactiplantibacillus pentosus T.

In one aspect of the present invention, the lactic acid bacterium of the genus Lactiplantibacillus may be a lactic acid bacterium other than Lactiplantibacillus plantarum.

### (4) Lactic Acid Bacteria of Genus Ligilactobacillus

Examples of lactic acid bacteria of the genus Ligilactobacillus include the following.
Ligilactobacillus salivarius
Ligilactobacillus acidipiscis

Preferable lactic acid bacteria of the genus Ligilactobacillus are Ligilactobacillus salivarius.

Ligilactobacillus salivarius may be any available bacterial strain and may include the type strain Ligilactobacillus salivarius T.

### (5) Lactic Acid Bacteria of Genus Lactococcus

Examples of lactic acid bacteria of the genus Lactococcus include the following. Lactococcus lactis subsp. lactis
Lactococcus lactis subsp. cremoris
Lactococcus lactis

Preferable lactic acid bacteria of the genus Lactococcus are Lactococcus lactis subsp. lactis or Lactococcus lactis subsp. cremoris.

Further preferable lactic acid bacteria of the genus Lactococcus are Lactococcus lactis subsp. lactis.

Lactococcus lactis subsp. lactis may be any available bacterial strain and may include the type strain Lactococcus lactis subsp. lactis T.

### (6) Streptococcus salivarius subsp. thermophilus

Streptococcus salivarius subsp. thermophilus may be any available bacterial strain and may include the type strain Streptococcus salivarius subsp. thermophilus T.

Mutants or derivatives of the specific bacterial strains mentioned above can also be used as long as they secrete outer membrane vesicles.

Preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum,
Ligilactobacillus salivarius,
Lactococcus lactis subsp. lactis, and
Streptococcus salivarius subsp. thermophilus.

In one aspect of the present invention, preferable lactic acid bacteria are at least one selected from the group consisting of the following:
Lactobacillus gasseri,
Lactobacillus amylovorus,
Lactobacillus crispatus,
Lactobacillus johnsonii,
Lacticaseibacillus casei, and
Lactiplantibacillus pentosus.

In another aspect of the present invention, preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum,
Ligilactobacillus salivarius,
Lactococcus lactis subsp. lactis,
Streptococcus salivarius subsp. thermophilus,
Lactobacillus gasseri,
Lactobacillus amylovorus,
Lactobacillus crispatus,
Lactobacillus johnsonii,
Lacticaseibacillus casei, and
Lactiplantibacillus pentosus.

More preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum,
Ligilactobacillus salivarius, and
Lactococcus lactis subsp. lactis.

In one aspect of the present invention, more preferable lactic acid bacteria are at least one selected from the group consisting of the following:
Lactobacillus gasseri,
Lactobacillus amylovorus,
Lactobacillus crispatus,
Lactobacillus johnsonii,
Lacticaseibacillus casei, and
Lactiplantibacillus pentosus.

In another aspect of the present invention, more preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum,
Ligilactobacillus salivarius,
Lactococcus lactis subsp. lactis,
Lactobacillus gasseri,
Lactobacillus amylovorus,
Lactobacillus crispatus,
Lactobacillus johnsonii,
Lacticaseibacillus casei, and
Lactiplantibacillus pentosus.

Further preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum, and
Ligilactobacillus salivarius.

In one aspect of the present invention, further preferable lactic acid bacteria are at least one selected from the group consisting of the following:
Lactobacillus gasseri,
Lactobacillus amylovorus,
Lactobacillus crispatus,
Lactobacillus johnsonii,
Lacticaseibacillus casei, and
Lactiplantibacillus pentosus.

In another aspect of the present invention, further preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum,
Ligilactobacillus salivarius,
Lactobacillus gasseri,
Lactobacillus amylovorus,
Lactobacillus crispatus,
Lactobacillus johnsonii,
Lacticaseibacillus casei, and
Lactiplantibacillus pentosus.

Still further preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei, and
Ligilactobacillus salivarius.

In one aspect of the present invention, still further preferable lactic acid bacteria are at least one selected from the group consisting of the following:
Lactobacillus gasseri,
Lactobacillus amylovorus,
Lactobacillus crispatus,
Lactobacillus johnsonii, and
Lacticaseibacillus casei.

In another aspect of the present invention, still further preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Ligilactobacillus salivarius,
Lactobacillus gasseri,
Lactobacillus amylovorus,
Lactobacillus crispatus,
Lactobacillus johnsonii, and
Lacticaseibacillus casei.

Particularly preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus, and
Lacticaseibacillus paracasei.

In one aspect of the present invention, particularly preferable lactic acid bacteria are at least one selected from the group consisting of the following:
Lactobacillus gasseri,
Lactobacillus amylovorus,
Lactobacillus crispatus,
Lactobacillus johnsonii, and
Lacticaseibacillus casei.

In another aspect of the present invention, particularly preferable lactic acid bacteria are at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactobacillus gasseri,
Lactobacillus amylovorus,
Lactobacillus crispatus,
Lactobacillus johnsonii, and
Lacticaseibacillus casei.

Even more particularly preferable lactic acid bacteria are at least one selected from the group consisting of the following:
Lactobacillus acidophilus,
Lacticaseibacillus paracasei, and
Lactobacillus gasseri.

The most preferable lactic acid bacteria are at least one selected from the group consisting of the following:
Lactobacillus acidophilus and
Lacticaseibacillus paracasei.

A single strain of lactic acid bacterium may be used, or multiple strains may be used in combination. Therefore, outer membrane vesicles derived from two or more species of lactic acid bacteria can be used in the present invention.

### [Outer Membrane Vesicles Derived from Lactic Acid Bacteria]

Outer membrane vesicles derived from bacteria are substances secreted from bacteria.

Outer membrane vesicles derived from lactic acid bacteria are spherical structures composed of cell membranes (lipid bilayer membranes) of lactic acid bacteria, and their diameter is generally about 20 to 500 nm. The spherical structures may contain nucleic acids (DNA and RNA) and proteins (such as enzymes). Outer membrane vesicles derived from cell membranes of lactic acid bacteria may also contain membrane proteins, peptidoglycans (PG), and the like present in the lipid bilayer membranes of lactic acid bacteria (Journal of the Japanese Society of Lactic Acid Bacteria, 27(1), 10-16, 2016, and Microbiol Spectr., 7(1)., 2019).

Outer membrane vesicles derived from bacteria are also referred to as membrane vesicles, extracellular vesicles (EV), extracellular vesicles, microvesicles, membrane vesicles (MV), outer membrane vesicles (OMV), and the like.

No special culture conditions are required for the secretion of outer membrane vesicles, and outer membrane vesicles are released into the medium by culturing under conditions generally used for lactic acid bacterial culture.

The medium preferably contains a carbon source, a nitrogen source, inorganic salts, and the like, so that lactic acid bacteria can be efficiently cultured. The medium may be a natural medium or a synthetic medium, and a known medium suitable for the bacterial strain to be used can be appropriately selected.

Examples of the carbon source include lactose, glucose, sucrose, fructose, galactose, and waste molasses.

Examples of the nitrogen source include organic nitrogen-containing substances, such as casein hydrolysates, whey protein hydrolysates, and soybean protein hydrolysates.

Examples of the inorganic salts include phosphates, sodium, potassium, and magnesium.

Examples of the medium suitable for lactic acid bacteria include MRS medium, GLM medium, GAM medium, BL medium, Briggs Liver Broth, animal milk, skim milk, and milk whey, with MRS medium and GLM medium being preferable.

When outer membrane vesicles of lactic acid bacteria are used for food purposes, a medium composed of only food materials and food additives can be used.

The lactic acid bacteria can be cultured, for example, at a temperature of 20 to 50°C (preferably 30 to 40°C) under static or anaerobic conditions. The culture temperature can be adjusted by a constant temperature bath, a mantle heater, a jacket, or the like.

The anaerobic condition refers to a low oxygen environment in which lactic acid bacteria can grow. Anaerobic conditions can be set using an anaerobic chamber, an anaerobic box, or a sealed container or bag containing an oxygen scavenger.

Examples of the culture format include static culture, shaking culture, and tank culture.

The culture time is, for example, 3 to 96 hours, preferably 12 to 32 hours.

The acquisition of outer membrane vesicles from a lactic acid bacterial culture solution can be achieved by appropriately combining one or more known separation and purification means based on the mass and size of the outer membrane vesicles.

Examples of the separation and purification means include centrifugation, ultracentrifugation, and filtration.

For example, outer membrane vesicles can be obtained using the following procedure.

The lactic acid bacterial culture solution is subjected to centrifugation (for example, centrifugal force: 3,000 to 15,000 g, preferably 8,000 to 10,000 g) to precipitate bacterial cells. The supernatant after centrifugation is filtered through a filter having a pore size (for example, 220 to 1,000 nm, preferably 450 nm (0.45 µm)) that allows outer membrane vesicles to pass through. The filtrate containing outer membrane vesicles is subjected to ultracentrifugation (for example, centrifugal force: 100,000 to 300,000 g, preferably 150,000 to 300,000 g) to obtain outer membrane vesicles as a precipitate. The precipitate may be further subjected to filtration.

### [FPR2 (Formyl Peptide Receptor 2)]

FPR2 (Formyl Peptide Receptor 2) is a G protein-coupled receptor and is a receptor for peptides having N-formylmethionine (for example, f-Met-Leu-Phe (FMLP)).

FPR2 is one of the FPR families (FPR1, FPR2, and FPR3) present in humans.

FPR2 is sometimes referred to as FPRL-1 or FPRL1.

FPR1 is called a formyl peptide receptor, and FPR2 and FPR3 are called formyl peptide-like receptors. FPR2 has higher sequence homology with FPR3 than with FPR1 (Pharmacol Rev 61:119-161, 2009).

### [FPR2 Agonist]

In the present invention, outer membrane vesicles derived from specific lactic acid bacteria are used to activate FPR2 (activation by binding to FPR2).

Therefore, one aspect of the present invention is an FPR2 agonist comprising outer membrane vesicles derived from specific lactic acid bacteria as an active ingredient. Furthermore, the use of outer membrane vesicles derived from specific lactic acid bacteria for preparing an FPR2 agonist is also one aspect of the present invention.

Although substances having agonist activity against FPR2, such as lowmolecular-weight compounds and peptides, are already known, it has not been reported so far that outer membrane vesicles derived from lactic acid bacteria activate FPR2. The present invention relates to an FPR2 agonist comprising outer membrane vesicles derived from specific lactic acid bacteria capable of activating FPR2. The outer membrane vesicles derived from lactic acid bacteria used as an active ingredient of the FPR2 agonist in the present invention are known to be very stable and have a property of being able to remain in the body for a long time without being completely decomposed even after being taken up by cells; therefore, unlike conventional substances having agonist activity against FPR2, the outer membrane vesicles themselves are considered to have both the FPR2 agonistic function and the function of stably delivering themselves into the body. In addition, as described above, outer membrane vesicles derived from lactic acid bacteria can be easily obtained by culturing lactic acid bacteria.

FPR2 agonists have been demonstrated to be beneficial in preclinical models of chronic inflammatory human diseases, including chronic inflammation resolution, infectious diseases, psoriasis, dermatitis, inflammatory bowel syndrome, Crohn's disease, ocular inflammation, sepsis, pain, metabolism/diabetes, cancer, COPD, asthma and allergic diseases, cystic fibrosis, acute lung injury and fibrosis, rheumatoid arthritis and other joint diseases, Alzheimer's disease, renal fibrosis, and organ transplantation (Patent Literature 7 and Non Patent Literatures 12 and 13); therefore, the outer membrane vesicles derived from specific lactic acid bacteria of the present invention are considered to be useful for the treatment of diseases associated with FPR2.

FPR2 agonistic activity can be measured using a known assay system (for example, a reporter assay described in International Publication No. WO2020/026979) capable of measuring activation by a ligand of a G protein-coupled receptor (GPCR). This reporter assay is provided as a commissioned service (for example, "Tanso Biosciences' GPCR Commissioned Test" in partnership with Cosmo Bio Co., Ltd.).

The outer membrane vesicles of lactic acid bacteria according to the present invention preferably activate FPR2 selectively.

The phrase "selectively activate FPR2" means having agonistic activity against FPR2 but not having agonistic activity against FPR1. Since FPR1 is associated with the induction of inflammatory responses (Non Patent Literature 3), an FPR2 agonist that "selectively activates FPR2" can treat diseases associated with FPR2 without inducing inflammatory responses.

### [Diseases Associated with FPR2]

FPR2 agonists can be used to treat diseases associated with FPR2.

"Diseases associated with FPR2" include not only those that are targets of pharmaceuticals but also those that are targets of functional foods and/or drinks and functional cosmetics.

"Treatment" includes not only acts intended for treatment and prevention (use of an FPR2 agonist as a pharmaceutical or quasi-drug) but also acts intended for health promotion (use of an FPR2 agonist as a functional food and/or drink or a functional cosmetic).

FPR2 agonists can be applied to both therapeutic use (medical use) and non-therapeutic use (non-medical use).

Examples of "diseases associated with FPR2" include the following.

Inflammatory diseases, heart disease, chronic airway disease, cancer, sepsis, allergy symptoms, circulatory disorders, neuroinflammation, neuropathy, pain, prion diseases, amyloidosis, immune disorders, Behcet's disease, Sweet's disease, systemic lupus erythematosus (SLE), Wegener's granulomatosis, viral infection, diabetes, bacterial infection, physical disorders including physical trauma and radiation exposure, vasoconstriction, anaphylactic reaction, allergic reaction, rhinitis, shock (endotoxic, hemorrhagic, traumatic, visceral ischemic, and circulatory shock), rheumatoid arthritis, gout, benign prostatic hyperplasia, myocardial ischemia, myocardial infarction, heart failure, brain injury, lung disease, COPD, COAD, COLD, acute lung injury, acute respiratory distress syndrome, chronic bronchitis, emphysema, asthma (allergic and nonallergic asthma), cystic fibrosis, renal fibrosis, renal impairment, renal glomerular disease, ulcerative colitis, IBD, Crohn's disease, periodontitis, pain, Alzheimer's disease, AIDS, uveitis glaucoma, conjunctivitis, Sjogren's syndrome, atherosclerosis, neuroinflammation including multiple sclerosis, seizures, skin diseases (rosacea, sunburn, psoriasis, hot flashes, facial flushing and redness associated with hot flashes, erythema associated with hot flashes, UV aging, seborrheic dermatitis, acne, allergic dermatitis, telangiectasia, rhinophyma, skin rash, skin erythema, skin hyperactivity with dilation of skin blood vessels, Lyell's syndrome, Stevens-Johnson syndrome, hemorrhoid-associated local itching and discomfort, hemorrhoids, erythema multiforme minor, erythema multiforme major, erythema nodosum, eye swelling, urticaria, pruritus, purpura, varicose veins, contact dermatitis, atopic dermatitis, nummular dermatitis, exfoliative dermatitis, stasis dermatitis, lichen simplex chronicus, perioral dermatitis, pseudofolliculitis barbae, granuloma annulare, actinic keratosis, basal cell carcinoma, squamous cell carcinoma, eczema, skin wound healing, hypertrophic scar, keloid, burn, actinic keratosis, melanoma, viral wart, aging caused by ultraviolet rays, photodamage, melanoderma, postinflammatory hyperpigmentation, other hyperpigmentation disorders, alopecia), ocular inflammation, enteritis, inflammatory bowel syndrome, dermatitis, tonsillitis, laryngitis, pharyngitis, sinusitis, esophagitis, gastritis, and the like.

The diseases mentioned above can be classified, for example, as follows.
(1) Inflammatory diseases (for example, tonsillitis, laryngitis, pharyngitis, ocular inflammation, rhinitis, sinusitis, periodontitis, conjunctivitis, neuroinflammation, chronic bronchitis, Behcet's disease, etc.)
(2) Digestive diseases (for example, esophagitis, gastritis, enteritis, inflammatory bowel syndrome, ulcerative colitis, etc.)
(3) Skin diseases (for example, dermatitis (including allergic dermatitis, atopic dermatitis, and seborrheic dermatitis), acne, rash, sunburn, hot flushes, eczema, aging caused by ultraviolet rays, itching and discomfort, perioral dermatitis, hyperpigmentation disorders, alopecia, etc.)
(4) Brain diseases (for example, dementia (including alcoholic dementia), migraine, Alzheimer's disease (including initial symptoms such as forgetfulness), prion diseases, etc.)
(5) Other diseases (respiratory diseases (for example, asthma, chronic obstructive pulmonary disease (COPD), emphysema, etc.), circulatory diseases (for example, atherosclerosis, heart disease, circulatory disorders, myocardial ischemia, myocardial infarction, heart failure, etc.), joint diseases (for example, rheumatoid arthritis, etc.), neurological diseases (for example, neuritis, neuropathy, pain, etc.), metabolic diseases (for example, diabetes, gout, renal impairment, etc.), infectious diseases (for example, bacterial infection, viral infection, sepsis caused by infection, etc.), immune disorders (for example, allergy symptoms, AIDS, etc.), and others (for example, physical trauma, cancer, Crohn's disease, etc.)).

FPR2 is known to be involved in brain neurotoxicity, intestinal epithelial cell homeostasis and inflammation, and proliferation and regeneration (Non Patent Literatures 8 to 11 mentioned previously). Epithelial cells are also present in the skin. In addition, outer membrane vesicles can pass through the blood-brain barrier (Non Patent Literatures 5 and 6). Therefore, FPR2 agonists are preferably used for the treatment of brain diseases (for example, Alzheimer's disease (including initial symptoms such as forgetfulness), dementia (including alcoholic dementia), and migraine), digestive diseases (for example, esophagitis, gastritis, enteritis, inflammatory bowel syndrome, and ulcerative colitis), and skin diseases (for example, dermatitis (including allergic dermatitis, atopic dermatitis, and seborrheic dermatitis), acne, rash, sunburn, hot flushes, eczema, aging caused by ultraviolet rays, itching and discomfort, perioral dermatitis, hyperpigmentation disorders, and alopecia) by binding to and activating FPR2.

The content of outer membrane vesicles derived from lactic acid bacteria in an FPR2 agonist can be appropriately set according to the condition, symptoms, etc. of the subject to which the agonist is applied.

The content (number) of outer membrane vesicles per mL of FPR2 agonist is, for example, 2×10³ to 2×10¹⁴ vesicles/mL, preferably 2×10⁴ to 2×10¹² vesicles/mL, and more preferably 2×10⁵ to 2×10¹⁰ vesicles/mL.

The FPR2 agonist can be applied without limitation to animals expressing FPR2. The subject is preferably a mammal (human and non-human mammal (for example, horse or cow)), more preferably human. The sex and age of the subject are not limited.

### [Induction of IL-10]

In the present invention, outer membrane vesicles derived from specific lactic acid bacteria are used to induce the production and secretion of IL-10 (hereinafter also referred to as "IL-10 induction"). This aspect can be understood as an IL-10 inducer containing outer membrane vesicles of lactic acid bacteria according to the present invention as an active ingredient.

In one aspect of the present invention, outer membrane vesicles induce IL-10 via activation of FPR2. This aspect can be understood as an IL-10 inducer by FPR2 activation containing outer membrane vesicles of lactic acid bacteria according to the present invention as an active ingredient.

IL-10 is an anti-inflammatory cytokine produced and secreted by immune cells such as macrophages, helper T cells, and regulatory T cells.

IL-10 inducers (including embodiments that induce IL-10 via FPR2 activation; the same applies hereinafter) can be used to treat diseases associated with IL-10.

"Diseases associated with IL-10" include not only those that are targets of pharmaceuticals but also those that are targets of functional foods and/or drinks and functional cosmetics.

"Treatment" includes not only acts intended for treatment and prevention (use of an IL-10 inducer as a pharmaceutical or quasi-drug) but also acts intended for health promotion (use of an IL-10 inducer as a functional food and/or drink or a functional cosmetic).

IL-10 inducers can be applied to both therapeutic use (medical use) and non-therapeutic use (non-medical use).

Examples of "diseases associated with IL-10" include (1) inflammatory diseases, (2) digestive diseases, (3) skin diseases, (4) brain diseases, and (5) other diseases described above.

The content of outer membrane vesicles derived from lactic acid bacteria in an IL-10 inducer can be appropriately set according to the condition, symptoms, etc. of the subject to which the IL-10 inducer is applied.

The content (number) of outer membrane vesicles per mL of IL-10 inducer is, for example, 2×10³ to 2×10¹⁴ vesicles/mL, preferably 2×10⁴ to 2×10¹² vesicles/mL, and more preferably 2×10⁵ to 2×10¹⁰ vesicles/mL.

The IL-10 inducer can be applied without limitation to animals. The subject is preferably a mammal (human and non-human mammal (for example, horse or cow)), more preferably human. The sex and age of the subject are not limited.

### [Protection of the Skin from Ultraviolet Rays]

In the present invention, outer membrane vesicles derived from specific lactic acid bacteria are used to protect the skin from ultraviolet rays. This aspect can be understood as a skin protectant against ultraviolet rays containing outer membrane vesicles of lactic acid bacteria according to the present invention as an active ingredient.

In one aspect of the present invention, outer membrane vesicles protect the skin from ultraviolet rays via activation of FPR2. This aspect can be understood as a skin protectant against ultraviolet rays by FPR2 activation containing outer membrane vesicles of lactic acid bacteria according to the present invention as an active ingredient.

Skin protectants against ultraviolet rays (including embodiments that protect the skin via FPR2 activation; the same applies hereinafter) can be used to treat skin diseases associated with ultraviolet rays.

"Skin diseases associated with ultraviolet rays" include not only those that are targets of pharmaceuticals but also those that are targets of functional foods and/or drinks and functional cosmetics.

"Treatment" includes not only acts intended for treatment and prevention (use of a skin protectant as a pharmaceutical or quasi-drug) but also acts intended for health promotion (use of a skin protectant as a functional food and/or drink or a functional cosmetic).

Skin protectants can be applied to both therapeutic use (medical use) and non-therapeutic use (non-medical use).

Examples of "skin diseases associated with ultraviolet rays" include (3) skin diseases described above.

The content of outer membrane vesicles derived from lactic acid bacteria in a skin protectant can be appropriately set according to the condition, symptoms, etc. of the subject to which the skin protectant is applied.

The content (number) of outer membrane vesicles per mL of skin protectant is, for example, 2×10³ to 2×10¹⁴ vesicles/mL, preferably 2×10⁴ to 2×10¹² vesicles/mL, and more preferably 2×10⁵ to 2×10¹⁰ vesicles/mL.

The skin protectant can be applied without limitation to animals. The subject is preferably a mammal (human and non-human mammal (for example, horse or cow)), more preferably human. The sex and age of the subject are not limited.

### [Protection of Neuronal Cells from Neurocytotoxicity]

In the present invention, outer membrane vesicles derived from specific lactic acid bacteria are used to protect neuronal cells from neurocytotoxicity. This aspect can be understood as a neuronal cell protectant against neurocytotoxicity containing outer membrane vesicles of lactic acid bacteria according to the present invention as an active ingredient.

In one aspect of the present invention, outer membrane vesicles protect neuronal cells from neurocytotoxicity via activation of FPR2. This aspect can be understood as a neuronal cell protectant against neurocytotoxicity by FPR2 activation containing outer membrane vesicles of lactic acid bacteria according to the present invention as an active ingredient.

Examples of neurocytotoxicity include neurocytotoxicity caused by endogenous toxins (amyloid β, oxygen radicals, etc.) and neurocytotoxicity caused by exogenous toxins (ethanol, etc.).

Neuronal cell protectants against neurocytotoxicity (including embodiments that protect neuronal cells via FPR2 activation; the same applies hereinafter) can be used to treat diseases associated with neurocytotoxicity.

"Diseases associated with neurocytotoxicity" include not only those that are targets of pharmaceuticals but also those that are targets of functional foods and/or drinks and functional cosmetics.

"Treatment" includes not only acts intended for treatment and prevention (use of a neuronal cell protectant as a pharmaceutical or quasi-drug) but also acts intended for health promotion (use of a neuronal cell protectant as a functional food and/or drink or a functional cosmetic).

Neuronal cell protectants can be applied to both therapeutic use (medical use) and non-therapeutic use (non-medical use).

Examples of "diseases associated with neurocytotoxicity" include (4) brain diseases described above.

The content of outer membrane vesicles derived from lactic acid bacteria in a neuronal cell protectant can be appropriately set according to the condition, symptoms, etc. of the subject to which the neuronal cell protectant is applied.

The content (number) of outer membrane vesicles per mL of neuronal cell protectant is, for example, 2×10³ to 2×10¹⁴ vesicles/mL, preferably 2×10⁴ to 2×10¹² vesicles/mL, and more preferably 2×10⁵ to 2×10¹⁰ vesicles/mL.

The neuronal cell protectant can be applied without limitation to animals. The subject is preferably a mammal (human and non-human mammal (for example, horse or cow)), more preferably human. The sex and age of the subject are not limited.

### [Optional Components]

The FPR2 agonist may contain one or more optional components as long as the effects of the active ingredient are not impaired. The optional components can be appropriately selected according to the usage mode of the FPR2 agonist and the like.

The optional components may be additives used in pharmaceuticals, cosmetics, foods and/or drinks, and the like.

Examples of the additives include: oils and fats (vegetable oils (soybean oil, corn oil, safflower oil, olive oil, etc.) and animal oils and fats (beef tallow, sardine oil, etc.)); crude drugs (royal jelly, ginseng, etc.); amino acids (glutamine, cysteine, leucine, arginine, etc.); polyhydric alcohols (ethylene glycol, polyethylene glycol, propylene glycol, glycerin, and sugar alcohols (sorbitol, erythritol, xylitol, maltitol, mannitol, etc.)); natural polymers (gum arabic, agar, soluble corn fiber, gelatin, xanthan gum, casein, gluten or gluten hydrolysate, lecithin, starch, dextrin, etc.); vitamins (vitamin C, vitamin B complex, etc.); minerals (calcium, magnesium, zinc, iron, etc.); dietary fiber (mannan, pectin, hemicellulose, etc.); surfactants (glycerin fatty acid esters, sorbitan fatty acid esters, etc.); purified water; excipients (glucose, cornstarch, lactose, dextrin, etc.); stabilizers; pH adjusters; antioxidants; sweeteners; flavoring agents; acidulants; colorants; fragrances, etc.

In addition, the FPR2 agonist may optionally contain functional components other than the active ingredient (outer membrane vesicles derived from lactic acid bacteria), such as taurine, glutathione, carnitine, creatine, coenzyme Q, glucuronic acid, glucuronolactone, capsicum extract, ginger extract, cacao extract, guarana extract, garcinia extract, theanine, γ-aminobutyric acid, capsaicin, capsiate, various organic acids, flavonoids, polyphenols, catechins, xanthine derivatives, indigestible oligosaccharides such as fructooligosaccharides, and polyvinylpyrrolidone.

The optional components are known substances and are readily available in the market or preparable.

A single species of optional component may be used, or multiple species may be used in combination.

The content of the optional components can be appropriately set according to the purpose of the blending and the like.

The description above regarding the optional components is also applied to the IL-10 inducers, skin protectants, and neuronal cell protectants according to the present invention.

### [Pharmaceutical Composition]

The outer membrane vesicles derived from specific lactic acid bacteria of the present invention can be used as pharmaceuticals or quasi-drugs. Therefore, one aspect of the present invention is a pharmaceutical composition comprising outer membrane vesicles derived from lactic acid bacteria as an active ingredient. Furthermore, the use of outer membrane vesicles of lactic acid bacteria for preparing a pharmaceutical composition is also one aspect of the present invention.

The pharmaceutical composition can be used for the treatment of the "diseases associated with FPR2" described above.

The pharmaceutical composition can be used as an active ingredient in a method of treating a "disease associated with FPR2," comprising administering an effective amount to a subject to be treated for the "disease associated with FPR2" described above.

The dosage form of the pharmaceutical composition is not particularly limited. Examples of the dosage form include oral preparations (tablets, capsules, granules, powders, fine granules, syrups, dry syrups, solutions, suspensions, inhalants, etc.); enteral preparations (suppositories, etc.); infusions; injections; and external preparations (aerosols, solutions, emulsions, creams, ointments, gels, lotions, poultices, tapes, etc.). Among these, oral preparations and external preparations are preferable.

Liquid preparations, such as solutions and suspensions, may be in a form that is dissolved or suspended in water or another suitable medium immediately before taking.

Tablets, granules, and the like may have their surfaces coated by a known method.

The pharmaceutical composition may be a sustained-release preparation, a delayed-release preparation, or an immediate-release preparation prepared according to known release control techniques.

The pharmaceutical composition may optionally contain pharmaceutically acceptable carriers or additives.

Examples of pharmaceutically acceptable carriers and additives include water, organic solvents, collagen, polyvinyl alcohol, polyvinylpyrrolidone, carboxyvinyl polymer, sodium alginate, water-soluble dextran, water-soluble dextrin, sodium carboxymethyl starch, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, lactose, surfactants, and artificial cell structures (such as liposomes).

The pharmaceutical composition can be applied without limitation to animals expressing FPR2. The subject is preferably a mammal (human and non-human mammal (for example, horse or cow)), more preferably human. The sex and age of the subject are not limited.

The dosage of the pharmaceutical composition can be appropriately set according to the type of target disease, age and weight of the administration target, administration route, number of administrations, administration purpose, and the like.

When administered orally, the number of outer membrane vesicles per kg of body weight of the subject is, for example, 2×10⁶ to 2×10¹⁴ vesicles/kg, preferably 2×10⁸ to 2×10¹³ vesicles/kg.

The administration interval can be appropriately set according to the type of target disease, etc., and may be once a day, or may be administered in several divided doses.

The above description regarding the pharmaceutical composition is also applied to the aspects of the present invention relating to the treatment of "diseases associated with IL-10," "skin diseases associated with ultraviolet rays," and "diseases associated with neurocytotoxicity."

### [Cosmetic Composition]

The outer membrane vesicles derived from specific lactic acid bacteria of the present invention can be used as an active ingredient of a cosmetic composition. Therefore, one aspect of the present invention is a cosmetic composition comprising outer membrane vesicles derived from lactic acid bacteria as an active ingredient.

The cosmetic composition of the present invention is a cosmetic composition in which the outer membrane vesicles derived from specific lactic acid bacteria of the present invention activate FPR2 to exert a certain function (for example, anti-inflammatory action, epithelial cell regeneration and repair action, etc.) on a living body, that is, a cosmetic composition for FPR2 activation.

The cosmetic composition may optionally contain, in addition to outer membrane vesicles derived from lactic acid bacteria, additives that are acceptable for blending into cosmetic compositions. Examples of the additives include adjuvants and carriers, such as antioxidants, stabilizers, solubilizers, vitamins, pigments, and fragrances.

Examples of product forms of the cosmetic composition include cosmetics, such as lotions, various creams, essences, and foundations, and facial cleansers, soaps, shampoos, treatments, and cosmetic serums. Examples of dosage forms of the cosmetic composition include lotions, creams, essences, soaps, shampoos, treatments, foams, and foundations.

The content of outer membrane vesicles in the cosmetic composition can be appropriately set in consideration of the form of the cosmetic composition and the required functions. The content (number) of outer membrane vesicles per mL of cosmetic composition is, for example, 2×10³ to 2×10¹⁴ vesicles/mL, preferably 2×10⁴ to 2×10¹² vesicles/mL.

The description above regarding the cosmetic composition is also applied to cases where the IL-10 inducers, skin protectants, and neuronal cell protectants according to the present invention are used as cosmetic compositions.

### [Foods and/or Drinks]

The outer membrane vesicles derived from specific lactic acid bacteria of the present invention can be used as foods and/or drinks (foods and beverages). Therefore, one aspect of the present invention is a food and/or drink containing outer membrane vesicles derived from lactic acid bacteria. Foods and/or drinks include all foods and/or drinks to which outer membrane vesicles derived from lactic acid bacteria can be added.

Specific examples of foods and/or drinks include functional foods and/or drinks (including those in various forms, such as tablet-shaped candies, tablets, chewable tablets, tablets, powders, fine powders, capsules, granules, and drinks); beverages (tea beverages (green tea, oolong tea, black tea, etc.), soft drinks, jelly drinks, sports drinks, milk beverages, carbonated drinks, vegetable drinks, fruit drinks, fermented vegetable drinks, fermented fruit drinks, fermented milk drinks (yogurt, etc.), lactic acid bacteria beverages, milk beverages (coffee milk, fruit milk, etc.), powder beverages, cocoa beverages, milk, and purified water, etc.); spreads (butter, jam, Japanese rice seasonings, margarine, etc.); mayonnaise; shortening; custard cream; dressings; breads; cooked rice; noodles; pasta; miso soup; tofu; yogurt; soups or sauces; confectionery (biscuits, cookies, chocolate, candy, cakes, ice cream, chewing gum, tablets, etc.); and liquid foods (such as enteral nutrition formula). Among these, functional foods and/or drinks are preferable.

Functional foods and/or drinks refer to foods and/or drinks in which the outer membrane vesicles derived from specific lactic acid bacteria of the present invention activate FPR2 to exert a certain function (for example, anti-inflammatory action, epithelial cell regeneration and repair action, brain function improving action, etc.) on a living body, that is, foods and/or drinks for FPR2 activation.

Functional foods and/or drinks include all so-called health foods and/or drinks. Examples of functional foods and/or drinks include foods with health claims (including foods for specified health use (including foods for specified health use with conditions), foods with functional claims, and foods with nutrient functional claims), foods and/or drinks for special dietary use, dietary supplement foods and/or drinks, health supplement foods and/or drinks, supplements (including various dosage forms, such as tablets, coated tablets, sugar-coated tablets, capsules, and liquids), and beauty foods and/or drinks (such as diet foods and/or drinks).

Functional foods and/or drinks include health foods and/or drinks to which health claims based on the food standards of the Codex Alimentarius (by the Codex Alimentarius Commission) are applied.

The foods and/or drinks may optionally contain, in addition to outer membrane vesicles derived from lactic acid bacteria, additives that are acceptable for blending into foods and/or drinks.

Examples of the additives include color developers (sodium nitrite, etc.); colorants (gardenia pigments, Red 102, etc.); flavors (orange flavors, etc.); sweeteners (stevia, aspartame, etc.); preservatives (sodium acetate, sorbic acid, etc.); emulsifiers (sodium chondroitin sulfate, propylene glycol fatty acid ester, etc.); antioxidants (disodium EDTA, vitamin C, etc.); pH adjusters (citric acid, etc.); chemical seasonings (sodium inosinate, etc.); thickeners (xanthan gum, etc.); leavening agents (calcium carbonate, etc.); antifoaming agents (calcium phosphate); binders (sodium polyphosphate, etc.); nutritional enhancers (calcium enhancers, vitamin A, etc.); and excipients (water-soluble dextrin, etc.).

Foods and/or drinks containing outer membrane vesicles derived from lactic acid bacteria can be produced by means available in the food field.

For example, outer membrane vesicles prepared in a liquid, gel, solid, powder, or granular form may be blended into foods and/or drinks. Alternatively, outer membrane vesicles may be directly mixed, dissolved, or suspended in food and/or drink materials.

Outer membrane vesicles may be applied, coated, impregnated, or sprayed onto foods and/or drinks.

Outer membrane vesicles may be uniformly dispersed or unevenly distributed in foods and/or drinks.

In the case of functional foods and/or drinks, outer membrane vesicles may be encapsulated in capsules, edible films, edible coating agents, and the like.

In the case of functional foods and/or drinks, outer membrane vesicles may be molded into shapes, such as tablets, after adding appropriate excipients or the like.

The foods and/or drinks containing outer membrane vesicles may be further processed, and such processed products are also included in the scope of the present invention.

The content of outer membrane vesicles in foods and/or drinks can be appropriately set in consideration of the form of the foods and/or drinks, the required taste, and the like. In the case of a beverage, the content (number) of outer membrane vesicles per mL of the beverage is, for example, 2×10³ to 2×10¹⁴ vesicles/mL, preferably 2×10⁴ to 2×10¹² vesicles/mL.

The intake interval can be appropriately set according to the type of food and/or drink, etc., and may be once a day, or may be taken in several divided doses.

The description above regarding the foods and/or drinks is also applied to cases where the IL-10 inducers, skin protectants, and neuronal cell protectants according to the present invention are used as foods and/or drinks.

### [Feed]

The outer membrane vesicles derived from specific lactic acid bacteria of the present invention can also be used as animal feed.

Examples of the feed include feed for livestock (cows, pigs, etc.), racehorses, and pets (dogs, cats, etc.).

Since feed is almost the same as foods and/or drinks except that the target is non-human, the above description regarding foods and/or drinks is also applied to feed.

The description above regarding the feed is also applied to cases where the IL-10 inducers, skin protectants, and neuronal cell protectants according to the present invention are used as feed.

### [Consumer Product Composition]

The outer membrane vesicles derived from specific lactic acid bacteria of the present invention can be used as an active ingredient of a consumer product composition. Therefore, one aspect of the present invention is a consumer product composition comprising outer membrane vesicles derived from lactic acid bacteria as an active ingredient.

The consumer product composition of the present invention is a consumer product composition in which the outer membrane vesicles derived from specific lactic acid bacteria of the present invention activate FPR2 to exert a certain function (for example, anti-inflammatory action, epithelial cell regeneration and repair action, etc.) on a living body, that is, a consumer product composition for FPR2 activation.

Examples of the consumer product composition include deodorants, disinfectants, antiperspirants, fine fragrances such as perfumes, compositions for treating hard surfaces such as fabrics and floors, and products such as wipes, with deodorants and disinfectants being preferable. Examples of dosage forms of the consumer product composition include liquid, solid, semi-solid, gel, spray, foam, liquid film, and foam forms.

The consumer product composition may optionally contain, in addition to outer membrane vesicles derived from lactic acid bacteria, additives that are acceptable for blending into consumer product compositions. Examples of the additives include surfactants, chelating agents, dispersants, enzymes, enzyme stabilizers, brighteners, foam suppressants, dyes, fragrances, pigments, fabric softeners, and anti-caking agents.

The content of outer membrane vesicles in the consumer product composition can be appropriately set in consideration of the product form. The content (number) of outer membrane vesicles per mL of consumer product composition is, for example, 2×10³ to 2×10¹⁴ vesicles/mL, preferably 2×10⁴ to 2×10¹² vesicles/mL.

The description above regarding the consumer product composition is also applied to cases where the IL-10 inducers, skin protectants, and neuronal cell protectants according to the present invention are used as consumer product compositions.

### [Examples]

Next, the effects of the present invention will be specifically described with reference to Examples, but the present invention is not limited to the Examples.

### [Test Bacterial Strains]

Twenty-six species of bacteria shown in Table 1 were used.

**Table 1**

| No. | Bacterial Name | Strain Name |
|---|---|---|
| 1 | Lactobacillus delbrueckii subsp. bulgaricus T | JCM1002 |
| 2 | Lactobacillus helveticus T | JCM1120 |
| 3 | Lactobacillus acidophilus T | JCM1132 |
| 4 | Lactobacillus acidophilus | JCM1023 |
| 5 | Lactobacillus acidophilus | CP1613 |
| 20 | Lactobacillus gasseri T | JCM1131 |
| 21 | Lactobacillus gasseri | CP2305 |
| 22 | Lactobacillus amylovorus T | JCM1126 |
| 23 | Lactobacillus crispatus T | JCM1185 |
| 24 | Lactobacillus johnsonii T | JCM2012 |
| 25 | Lacticaseibacillus casei T | JCM1134 |
| 6 | Lacticaseibacillus rhamnosus T | JCM1136 |
| 7 | Lacticaseibacillus paracasei T | JCM8130 |
| 8 | Lacticaseibacillus paracasei | JCM1133 |
| 9 | Lacticaseibacillus paracasei | CP3526 |
| 10 | Lactiplantibacillus plantarum T | JCM1149 |
| 26 | Lactiplantibacillus pentosus T | NBRC106467 |
| 11 | Ligilactobacillus salivarius T | JCM1231 |
| 12 | Limosilactobacillus reuteri T | JCM1112 |
| 13 | Limosilactobacillus fermentum T | JCM1173 |
| 14 | Levilactobacillus brevis T | JCM1059 |
| 15 | Lactococcus lactis subsp. lactis T | JCM5805 |
| 16 | Streptococcus salivarius subsp. thermophilus T | JCM17834 |
| 17 | Escherichia coli T | JCM1649 |
| 18 | Escherichia coli | NBRC12734 |
| 19 | Escherichia coli | ATCC27166 |

| | | |
|---|---|---|
| Bacteria No. 1 to 14 and 20 to 26 were lactobacilli. Bacteria No. 15 to 16 were lactococci. Bacteria No. 17 to 19 were Escherichia coli. Bacteria No. 12 to 14 and 17 to 19 were used as Comparative Examples. | | |

### [Preparation Example: Preparation of Outer Membrane Vesicles Derived from Lactic Acid Bacteria]

### [Preparation of Outer Membrane Vesicles Derived from Lactobacilli]

(1) Lactobacilli were subjected to pre-culture using MRS liquid medium (manufactured by BD Japan) (static culture at 37°C for 16 hours).

The pre-culture solution was inoculated into MRS liquid medium (inoculation amount: 5% by volume) and subjected to main culture (static culture at 37°C for 16 hours).

The main culture solution was subjected to centrifugation (Tomy Seiko, MX-301, 10,000 g for 5 minutes).
(2) The supernatant after centrifugation was filtered through a polyvinylidene fluoride (PVDF) membrane filter (pore size: 0.45 µm).

The filtrate was subjected to ultracentrifugation (manufactured by Beckman Coulter, Optima XE-90, 150,000 g for 2 hours).

The precipitate obtained by removing the supernatant after ultracentrifugation was suspended in 2.5 mL of phosphate buffered saline (PBS) previously filtered through a PVDF membrane filter (pore size: 0.1 µm) to obtain an outer membrane vesicle suspension.

The outer membrane vesicle suspension was mixed with MRS liquid medium (manufactured by BD Japan) previously filtered through a PVDF membrane filter (pore size: 0.1 µm) at a volume ratio of 1:1.

The mixture was treated with ExoQuick-TC (registered trademark) ULTRA (manufactured by System Biosciences, LLC) and further filtered through a PVDF membrane filter (pore size: 0.45 µm) to obtain a purified outer membrane vesicle suspension.
(3) Based on the number of outer membrane vesicles determined according to the following quantification procedure, the purified outer membrane vesicle suspension was appropriately diluted with a buffer attached to ExoQuick-TC (registered trademark) ULTRA (manufactured by System Biosciences, LLC) to prepare a suspension having an outer membrane vesicle concentration of 6×10⁸ vesicles/mL. This suspension was stored frozen (-20°C) until used in the activity tests described below.

### [Quantification of Number of Outer Membrane Vesicles]

100 µL of a purified outer membrane vesicle suspension was diluted 10-fold with phosphate buffered saline (PBS) containing 5.56 µM of 3,3'-dioctadecyloxacarbocyanine perchlorate (biomembrane labeling fluorescent probe), and then subjected to a reaction in the dark (at 37°C for 2 hours) to label the outer membrane vesicles.

The reaction solution was filtered through a PVDF membrane filter (pore size: 0.45 µm), and the filtrate was diluted 100-fold with PBS.

The number of outer membrane vesicles in the diluted solution was measured using a "Small particle detection mode" of an ultra-sensitive high-end benchtop flow cytometer (Cell Stream, manufactured by Luminex).

In the measurement, the laser output at wavelengths of 488 nm and 642 nm was set to 100%, and the output of forward scattered light and side scattered light was set to 0%.

The number of particles detected at an excitation wavelength of 488 nm and a fluorescence wavelength of 528 nm was measured for 180 seconds, and the number of detected particles was defined as the number of outer membrane vesicles contained in the purified outer membrane vesicle suspension.

### [Preparation of Outer Membrane Vesicles Derived from Lactococci]

Lactococci were subjected to pre-culture using GLM17 medium (static culture at 30°C for 16 hours). The GLM17 medium was prepared by adding 0.5% by weight of glucose and 0.5% by weight of lactose to M17 liquid medium (manufactured by BD Japan).

The pre-culture solution was inoculated into GLM17 medium (inoculation amount: 5% by volume) and subjected to main culture (static culture at 30°C for 16 hours).

The main culture solution was subjected to centrifugation (Tomy Seiko, MX-301, 10,000 g for 5 minutes).

The supernatant after centrifugation was subjected to the procedures described in (2) and (3) of [Preparation of Outer Membrane Vesicles Derived from Lactobacilli] above to obtain a suspension having an outer membrane vesicle concentration of 6×10⁸ vesicles/mL. This suspension was stored frozen (-20°C) until used in the activity tests.

### [Preparation of Outer Membrane Vesicles Derived from Escherichia coli]

Escherichia coli was subjected to pre-culture using LB medium (manufactured by Kanto Chemical Co., Inc.) (shaking culture at 37°C for 16 hours).

The pre-culture solution was inoculated into LB medium (inoculation amount: 5% by volume) and subjected to main culture (shaking culture at 37°C for 16 hours).

The main culture solution was subjected to centrifugation (Tomy Seiko, MX-301, 8,000 g for 5 minutes).

The supernatant after centrifugation was subjected to the procedures described in (2) and (3) of [Preparation of Outer Membrane Vesicles Derived from Lactobacilli] above to obtain a suspension having an outer membrane vesicle concentration of 6×10⁸ vesicles/mL. This suspension was stored frozen (-20°C) until used in the activity tests.

### [Example 1: FPR2 Agonistic Activity]

The FPR2 agonistic activity of outer membrane vesicles was evaluated by "Tanso Biosciences' GPCR Commissioned Test" (in partnership with Cosmo Bio Co., Ltd.). In this test, a reporter assay employing the measurement principle described in International Publication No. WO2020/026979 was carried out.

An outer membrane vesicle suspension (final concentration of the number of outer membrane vesicles: 2×10⁶ vesicles/mL) prepared from a frozen suspension (described above) was used as an evaluation sample.

A blank sample was prepared according to the following procedure. MRS liquid medium (manufactured by BD Japan) filtered through a PVDF membrane filter (pore size: 0.1 µm) was mixed with phosphate buffered saline (PBS) filtered through a PVDF membrane filter (pore size: 0.1 µm) at a volume ratio of 1:1. The mixture was treated with ExoQuick-TC (registered trademark) ULTRA (manufactured by System Biosciences, LLC) to obtain a blank sample.

In the positive control sample, the peptide WKYMVM (Trp-Lys-Tyr-Met-Val-Met-NH2) (SEQ ID NO: 1) was used instead of outer membrane vesicles. This peptide is known to have FPR2 agonistic activity (Molecules 2017, 22, 455). A sample containing only the solvent used in the test was used as a negative control sample.

The test was carried out three times (sample size (n)=3).

The FPR2 agonistic rate was calculated according to the following formula. The results of the agonistic rate are shown as the average of the values calculated in three tests. FPR2 agonistic rate (%)=[(Reporter luminescence by test sample-Reporter luminescence by negative control sample)/(Reporter luminescence by positive control sample-Reporter luminescence by negative control sample)]×100

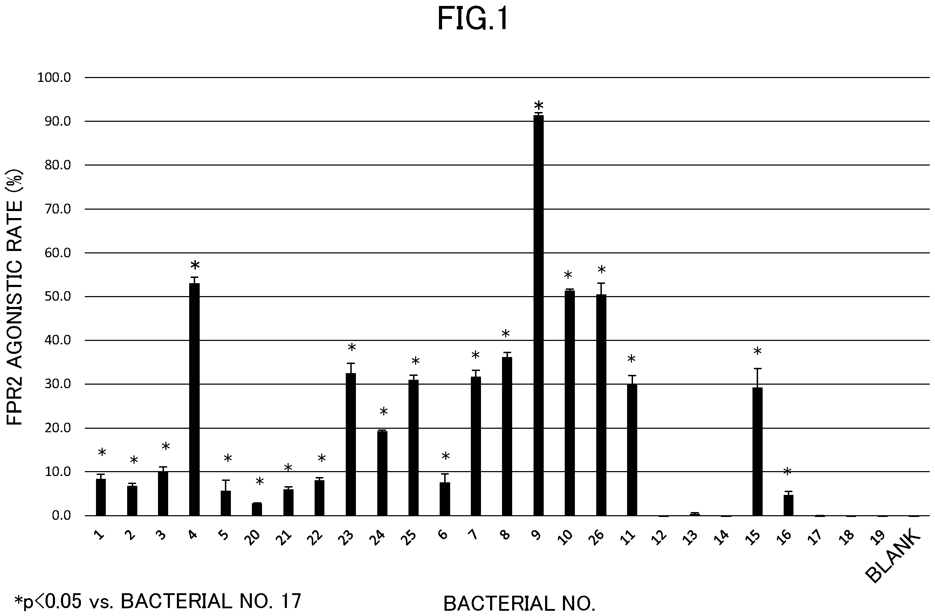

Student's t-test was used for statistical processing. The test was performed in comparison with the agonistic rate by outer membrane vesicles derived from Escherichia coli T (bacterial No. 17 in Table 1). A p-value<0.05 was considered statistically significant. The results are shown in Table 2 and Fig. 1.

**Table 2**

| Bacterial No. | FPR2 Agonistic Rate (%) | | |
|---|---|---|---|
| | Average | Standard Deviation | p-Value |
| 1 | 8.3 | 1.1 | 0.0004 |
| 2 | 6.8 | 0.6 | 0.0001 |
| 3 | 9.9 | 1.2 | 0.0003 |
| 4 | 53.0 | 1.4 | 0.0000 |
| 5 | 5.7 | 2.4 | 0.0264 |
| 20 | 2.8 | 0.1 | 0.0003 |
| 21 | 6.0 | 0.6 | 0.0002 |
| 22 | 8.1 | 0.6 | 0.0000 |
| 23 | 32.5 | 2.2 | 0.0000 |
| 24 | 19.2 | 0.2 | 0.0001 |
| 25 | 31.0 | 1.0 | 0.0000 |
| 6 | 7.6 | 2.0 | 0.0051 |
| 7 | 31.7 | 1.4 | 0.0000 |
| 8 | 36.1 | 1.2 | 0.0000 |
| 9 | 91.3 | 0.6 | 0.0000 |
| 10 | 51.4 | 0.1 | 0.0000 |
| 26 | 50.5 | 2.6 | 0.0000 |
| 11 | 30.0 | 1.9 | 0.0000 |
| 12 | -0.5 | 0.0 | 0.4800 |
| 13 | 0.4 | 0.2 | 0.0687 |
| 14 | -0.4 | 0.4 | 0.8132 |
| 15 | 29.2 | 4.4 | 0.0007 |
| 16 | 4.7 | 0.8 | 0.0013 |
| 17 | -0.3 | 0.4 | - |
| 18 | -0.6 | 0.0 | 0.4342 |
| 19 | -0.6 | 0.3 | 0.4360 |
| Blank | -1.0 | 0.3 | 0.1157 |

As shown in Table 2 and Fig. 1, the outer membrane vesicles derived from lactic acid bacteria according to the present invention (bacterial No. 1 to 11, 15 and 16, and 20 to 26) had FPR2 agonistic activity. On the other hand, outer membrane vesicles derived from lactic acid bacteria other than the lactic acid bacteria according to the present invention and outer membrane vesicles derived from Escherichia coli did not have FPR2 agonistic activity. Therefore, it was shown that among outer membrane vesicles derived from bacteria, the outer membrane vesicles derived from specific species of lactic acid bacteria according to the present invention are useful as FPR2 agonists.

### [Example 2: FPR1 Agonistic Activity]

The FPR1 agonistic activity of outer membrane vesicles was evaluated by "Tanso Biosciences' GPCR Commissioned Test" (in partnership with Cosmo Bio Co., Ltd.). In this test, a reporter assay employing the measurement principle described in International Publication No. WO2020/026979 was carried out.

An outer membrane vesicle suspension (final concentration of the number of outer membrane vesicles: 2×10⁶ vesicles/mL) prepared from a frozen suspension (described above) was used as an evaluation sample.

A blank sample was prepared according to the following procedure. MRS liquid medium (manufactured by BD Japan) filtered through a PVDF membrane filter (pore size: 0.1 µm) was mixed with phosphate buffered saline (PBS) filtered through a PVDF membrane filter (pore size: 0.1 µm) at a volume ratio of 1:1. The mixture was treated with ExoQuick-TC (registered trademark) ULTRA (manufactured by System Biosciences, LLC) to obtain a blank sample.

In the positive control sample, N-formylmethionyl-leucyl-phenylalanine (fMLP) was used instead of outer membrane vesicles. This peptide is known to have FPR1 agonistic activity (reference: Molecules 2017, 22, 455). A sample containing only the solvent used in the test was used as a negative control sample.

The test was carried out three times (sample size (n)=3).

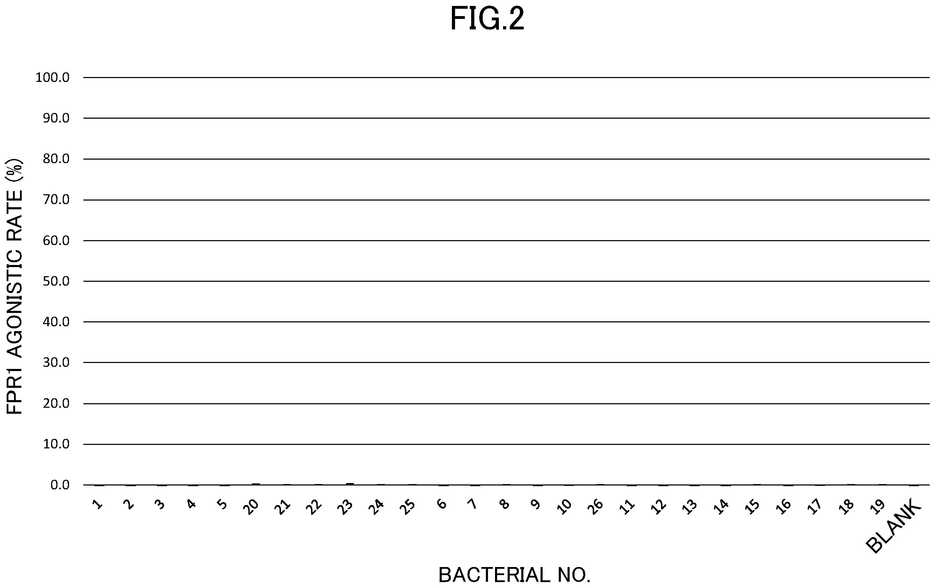

The FPR1 agonistic rate was calculated according to the following formula. The results of the agonistic rate are shown as the average of the values calculated in three tests. The results are shown in Table 3 and Fig. 2. FPR1 agonistic rate (%)=[(Reporter luminescence by test sample-Reporter luminescence by negative control sample)/(Reporter luminescence by positive control sample-Reporter luminescence by negative control sample)]×100

**Table 3**

| Bacterial No. | FPR1 Agonistic Rate (%) | |
|---|---|---|
| | Average | Standard Deviation |
| 1 | -0.4 | 0.0 |
| 2 | -0.3 | 0.0 |
| 3 | -0.3 | 0.2 |
| 4 | -0.6 | 0.1 |
| 5 | -0.7 | 0.0 |
| 20 | 0.1 | 0.1 |
| 21 | 0.0 | 0.0 |
| 22 | 0.0 | 0.0 |
| 23 | 0.1 | 0.2 |
| 24 | 0.0 | 0.0 |
| 25 | 0.0 | 0.0 |
| 6 | -0.7 | 0.1 |
| 7 | -0.7 | 0.1 |
| 8 | -0.1 | 0.1 |
| 9 | -0.2 | 0.1 |
| 10 | -0.2 | 0.0 |
| 26 | 0.0 | 0.0 |
| 11 | -0.7 | 0.2 |
| 12 | -0.5 | 0.1 |
| 13 | -0.2 | 0.0 |
| 14 | -0.5 | 0.0 |
| 15 | -0.1 | 0.1 |
| 16 | -0.8 | 0.2 |
| 17 | -0.2 | 0.1 |
| 18 | -0.1 | 0.1 |
| 19 | -0.1 | 0.1 |
| Blank | -0.6 | 0.2 |

As shown in Table 3 and Fig. 2, the outer membrane vesicles derived from lactic acid bacteria according to the present invention (bacterial No. 1 to 11, 15 and 16, and 20 to 26) did not have FPR1 agonistic activity. Therefore, it was shown that the outer membrane vesicles are useful as selective agonists of FPR2 because they selectively activate FPR2.

### [Reference Example 1: Effect of Known FPR2 Agonists on IL-10 Production by M2 Macrophages]

The anti-inflammatory cytokine IL-10 inducing ability in M2 macrophages differentiated from human monocyte-derived cells (THP-1, JCRB Cell Bank) was evaluated by the following procedure, which is a modification of the method described in the literature (BMC Cancer 15:577 (2015)).

THP-1 cells that had been subcultured in a medium (growth medium) prepared by adding 10% by volume of fetal bovine serum (hiFBS, Cytiva) inactivated at 56°C for 30 minutes and 1% by volume of penicillin-streptomycin solution (manufactured by FUJIFILM Wako Pure Chemical Corporation) to RPMI 1640 medium (manufactured by Nacalai Tesque, Inc.) were suspended in a medium prepared by adding 150 nM of phorbol 12-myristate 13-acetate (PMA, Sigma-Aldrich) to the growth medium so as to have a concentration of 1×10⁶ cells/mL, seeded in 96-well plates at 0.1 mL per well, and cultured at 37°C for 24 hours in the presence of 5% by volume of CO₂.

After the culture, the medium was removed, the cells were washed twice with the growth medium, 0.1 mL of the growth medium was added, and the cells were cultured at 37°C for 24 hours in the presence of 5% by volume of CO₂.

After the culture, the medium was removed, 0.1 mL of a medium prepared by adding 20 ng/mL of Human IL-4 (R&D Systems) and 20 ng/mL of Human IL-13 (Thermo Fisher Scientific) to the growth medium was added, and the cells were cultured at 37°C for 24 hours in the presence of 5% by volume of CO₂.

After the culture, the cells were washed twice with the growth medium, 0.1 mL of the growth medium was added, 2% by volume of a test substance (described below) was added, and the cells were cultured at 37°C for 24 hours in the presence of 5% CO₂.

After the culture, the culture supernatant was collected, and the IL-10 concentration in the supernatant was calculated using Human IL-10 DuoSet ELISA (R&D Systems).

It is known that the IL-10 inducing ability of M2 macrophages is induced by Toll-like receptor (TLR2) agonists (Arthritis Research & Therapy 19:245 (2017)). Therefore, three types of test samples were used, namely 500 ng/mL of FSL-1 (InvivoGen), which is a TLR2 agonist (FSL-1 group), a mixture of 500 ng/mL of FSL-1 and 5000 nM of WKYMVm (Trp-Lys-Tyr-Met-Val-D-Met-NH2 (SEQ ID NO: 2), Abcam), which is an FPR2 agonist (FSL-1+WKYMVm group), and a solvent alone (negative control group).

The test samples were added to the cells in a test state at 2% by volume. Therefore, the final concentration of the test sample at the time of the test was 1/50 of the concentration at the time of preparation.

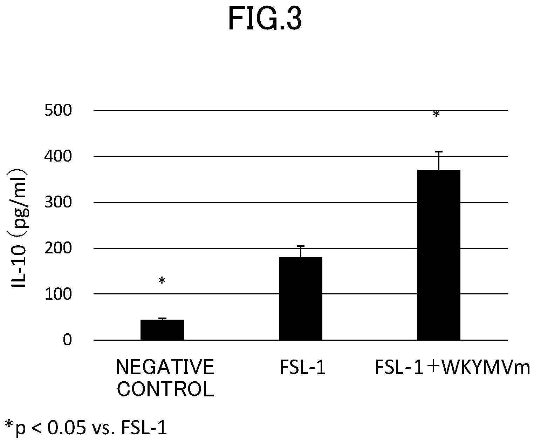

Student's t-test was used for statistical processing. The test was performed in comparison with the IL-10 concentration in the FSL-1 group. A p-value<0.05 was considered statistically significant. The sample size (n) was 6. The results of IL-10 concentration are shown as the average of the values measured in six tests. The results are shown in Table 4 and Fig. 3.

**Table 4**

| Group | IL-10 Concentration (pg/mL) | | |
|---|---|---|---|
| | Average | Standard Deviation | p-Value |
| Negative Control | 44.1 | 3.4 | 2.0E-07 |
| FSL-1 | 180.8 | 24.3 | - |
| FSL-1+WKYMVm | 368.9 | 41.1 | 5.0E-06 |

As shown in Table 4 and Fig. 3, co-treatment with FSL-1 and the FPR2 agonist induced secretion of the anti-inflammatory cytokine IL-10 at a significantly higher level compared to the single treatment with FSL-1, which is a TLR2 agonist. This indicated that the FPR2 agonistic activity is important in the anti-inflammatory action of immune cells.

### [Example 3: IL-10 Inducing Activity]

Purified outer membrane vesicle suspensions (test samples) derived from lactic acid bacteria were prepared according to the procedure described in [Preparation Example] above, except that the outer membrane vesicle concentration was set to 5×10⁸ vesicles/mL.

The IL-10 inducing activity of the test samples was evaluated according to the procedure described in [Reference Example 1] above. A blank sample was prepared by the method described in [Example 1] above. 500 ng/mL of FSL-1 (InvivoGen) was used as a positive control sample. A sample containing only the solvent used in the test was used as a negative control sample.

The test samples were added to the cells in a test state at 2% by volume. Therefore, the final concentration of outer membrane vesicles at the time of the test was 1/50 of the concentration at the time of preparation.

The IL-10 induction rate was calculated according to the following formula. IL-10 induction rate (%)=[(IL-10 concentration by addition of test sample - IL-10 concentration by addition of negative control sample)/(IL-10 concentration by addition of positive control sample - IL-10 concentration by addition of negative control sample)]×100

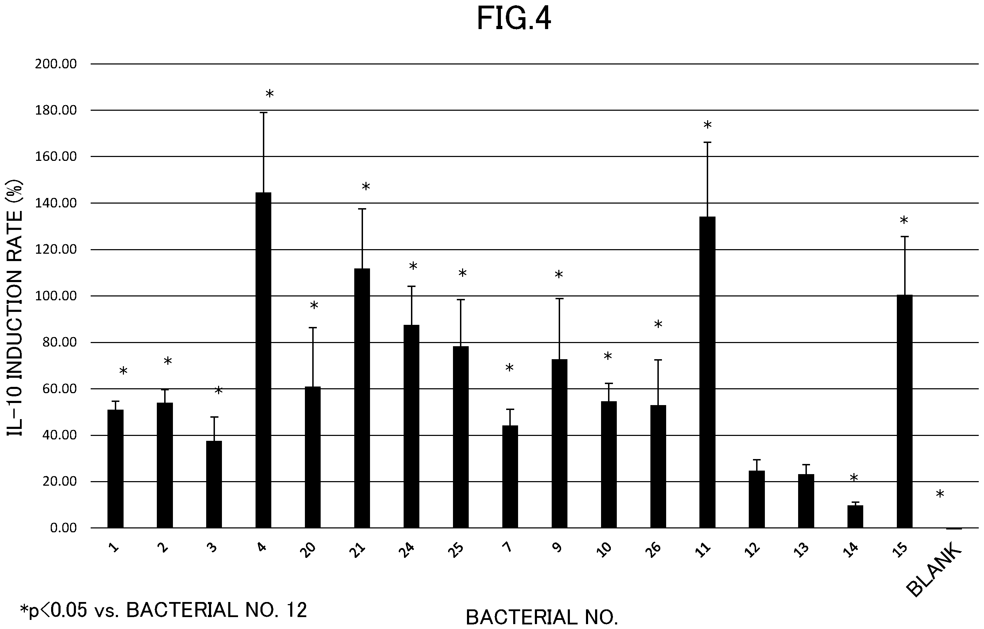

Student's t-test was used for statistical processing. The test was performed in comparison with the induction rate by outer membrane vesicles derived from Limosilactobacillus reuteri (bacterial No. 12 in Table 1). A p-value<0.05 was considered statistically significant. The sample size (n) was 6. The results of the IL-10 induction rate are shown as the average of the values calculated in six tests. The results are shown in Table 5 and Fig. 4.

**Table 5**

| Bacterial No. | IL-10 Induction Rate (%) | | |
|---|---|---|---|
| | Average | Standard Deviation | p-Value |
| 1 | 50.95 | 3.69 | 2.1E-06 |
| 2 | 53.94 | 5.69 | 5.1E-06 |
| 3 | 37.48 | 10.31 | 1.7E-02 |
| 4 | 144.47 | 34.59 | 1.7E-05 |
| 20 | 60.98 | 25.37 | 1.0E-02 |
| 21 | 111.83 | 25.64 | 2.1E-05 |
| 24 | 87.46 | 16.73 | 1.1E-05 |
| 25 | 78.28 | 20.20 | 1.8E-04 |
| 7 | 44.09 | 7.10 | 4.8E-04 |
| 9 | 72.59 | 26.24 | 2.4E-03 |
| 10 | 54.62 | 7.61 | 2.2E-05 |
| 26 | 52.93 | 19.50 | 1.0E-02 |
| 11 | 134.07 | 32.14 | 2.0E-05 |
| 12 | 24.59 | 4.84 | - |
| 13 | 23.22 | 4.09 | 6.4E-01 |
| 14 | 9.76 | 1.47 | 6.4E-05 |
| 15 | 100.35 | 25.26 | 6.2E-05 |
| Blank | -3.50 | 2.14 | 3.2E-07 |

As shown in Table 5 and Fig. 4, outer membrane vesicles derived from lactic acid bacteria having FPR2 agonistic activity (bacterial No. 1 to 4, 20, 21, 24, 25, 7, 9, 10, 26, 11, and 15 (Example 1)) showed a significantly higher IL-10 induction rate compared to outer membrane vesicles of lactic acid bacterium (bacterial No. 12) having no FPR2 agonistic activity. Therefore, it was shown that among outer membrane vesicles derived from bacteria, the outer membrane vesicles derived from specific species of lactic acid bacteria according to the present invention are useful as IL-10 inducers by FPR2 activation. This suggested that the FPR2 agonistic activity is important in the anti-inflammatory action of outer membrane vesicles derived from specific species of lactic acid bacteria according to the present invention.

### [Example 4: Effect of FPR2 Antagonist on IL-10 Inducing Ability of Outer Membrane Vesicles Having FPR2 Agonistic Activity]

A purified outer membrane vesicle suspension derived from Lacticaseibacillus paracasei strain CP3526 (bacterial No. 9) was prepared according to the procedure described in [Preparation Example] above, except that the outer membrane vesicle concentration was set to 2.5×10⁹ vesicles/mL.

Three types of test samples were used, namely outer membrane vesicles alone (CP3526 group), a mixture of outer membrane vesicles and 50000 nM of WRW4 (H-Trp-Arg-Trp-Trp-Trp-Trp-NH2 (SEQ ID NO: 3), Bachem AG), which is an FPR2 antagonist (CP3526+WRW4 group), and a solvent alone (negative control group).

The test samples were added to the cells in a test state at 2% by volume. Therefore, the final concentration of outer membrane vesicles at the time of the test was 1/50 of the concentration at the time of preparation.

The IL-10 concentration was evaluated according to the procedure described in [Reference Example 1] above.

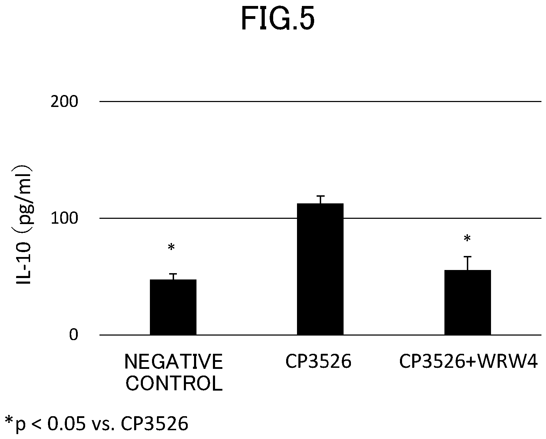

Student's t-test was used for statistical processing. The test was performed in comparison with the IL-10 concentration in the CP3526 group. A p-value<0.05 was considered statistically significant. The sample size (n) was 6. The results of IL-10 concentration are shown as the average of the values measured in six tests. The results are shown in Table 6 and Fig. 5.

**Table 6**

| Group | IL-10 Concentration (pg/mL) | | |
|---|---|---|---|
| | Average | Standard Deviation | p-Value |
| Negative Control | 47.61 | 4.79 | 2.0E-08 |
| CP3526 | 112.41 | 6.56 | - |
| CP3526+WRW4 | 55.55 | 11.66 | 1.4E-05 |

As shown in Table 6 and Fig. 5, the IL-10 concentration with addition of the FPR2 antagonist (CP3526+WRW4 group) was significantly lower than that without addition (CP3526 group). Therefore, it was shown that the outer membrane vesicles derived from Lacticaseibacillus paracasei strain CP3526 according to the present invention induce IL-10 via activation of FPR2. This suggested that the FPR2 agonistic activity is necessary for the anti-inflammatory action of outer membrane vesicles derived from specific species of lactic acid bacteria according to the present invention in immune cells.

### [Example 5: Action to Protect Skin from Ultraviolet Rays]

A purified outer membrane vesicle suspension derived from Lacticaseibacillus paracasei strain CP3526 (bacterial No. 9) was prepared according to the procedure described in [Preparation Example] above, except that the outer membrane vesicle concentration was set to 1.0×10¹⁰ vesicles/mL.

Three types of test samples were used, namely outer membrane vesicles alone (CP3526 group), a mixture of outer membrane vesicles and 50000 nM of WRW4 (Bachem AG), which is an FPR2 antagonist (CP3526+WRW4 group), and a solvent alone (negative control group).

Proliferated normal human epidermal keratinocytes (NHEK, KURABO INDUSTRIES LTD.) were suspended in HuMedia-KG2 (KURABO INDUSTRIES LTD.) at a concentration of 1.0×10⁵ cells/mL, seeded in 96-well black plates at 0.1 mL per well, and cultured at 37°C for 24 hours in the presence of 5% by volume of CO₂.

After the culture, the cells were washed with PBS, 0.02 mL of PBS was added, and the cells were irradiated with 75 mJ/cm² of UV-B.

After the irradiation, PBS was removed, and 0.1 mL of HuMedia-KG2 containing 2% by volume of the test sample was added. Therefore, the final concentration of outer membrane vesicles at the time of the test was 1/50 of the concentration at the time of preparation.

After addition of the test sample, the cells were cultured for 24 hours, and the number of viable cells was measured by the WST-8 assay.

The measurement by the WST-8 assay was as follows. The medium was replaced with HuMedia-KG2 containing 10% by volume of Cell Count Reagent SF (Nacalai Tesque), and the cells were incubated in a CO₂ incubator, and the amount of change in absorbance (450 nm) over 60 minutes from 30 minutes to 90 minutes after the incubation was measured. The cell viability was determined by dividing each value of the amount of change by the average value of the amount of change in absorbance in three tests without UV-B irradiation and multiplying the result by 100.

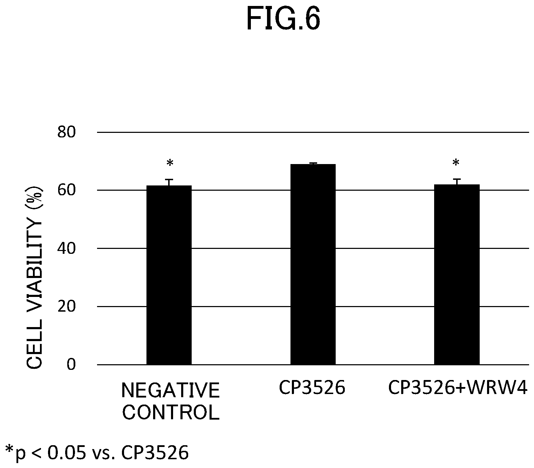

Student's t-test was used for statistical processing. The test was performed in comparison with the cell viability in the CP3526 group. A p-value<0.05 was considered statistically significant. The sample size (n) was 3. The results of cell viability are shown as the average of the values calculated in three tests. The results are shown in Table 7 and Fig. 6.

**Table 7**

| Group | Cell Viability (%) | | |
|---|---|---|---|
| | Average | Standard Deviation | p-Value |
| Negative Control | 61.58 | 2.08 | 0.03 |
| CP3526 | 68.96 | 0.40 | - |
| CP3526+WRW4 | 61.98 | 1.79 | 0.02 |

As shown in Table 7 and Fig. 6, addition of outer membrane vesicles significantly improved the viability of skin cells after ultraviolet irradiation compared to no addition. Furthermore, the viability was significantly lower with addition of the FPR2 antagonist than without addition. Therefore, it was shown that the outer membrane vesicles derived from Lacticaseibacillus paracasei strain CP3526 according to the present invention protect the skin from ultraviolet rays via activation of FPR2. This suggested that the FPR2 agonistic activity is necessary for the protective action of outer membrane vesicles derived from specific species of lactic acid bacteria according to the present invention in skin cells.

### [Example 6: Action to Protect Neuronal Cells from Neurocytotoxicity]

A purified outer membrane vesicle suspension derived from Lacticaseibacillus paracasei strain CP3526 (bacterial No. 9) was prepared according to the procedure described in [Preparation Example] above, except that the outer membrane vesicle concentration was set to 1.0×10¹⁰ vesicles/mL.

Two types of test samples were used, namely outer membrane vesicles alone (CP3526 group) and a solvent alone (negative control group).

Proliferated PC-12 cells (ECACC) were suspended at a concentration of 8×10⁴ cells/mL in a medium (growth induction medium) prepared by adding 5% by volume of fetal bovine serum (FBS, Thermo Fisher Scientific), 10% by volume of Horse Serum (hiHS, Thermo Fisher Scientific) inactivated at 56°C for 30 minutes, and 1% by volume of penicillin-streptomycin solution (P/S) (Nacalai Tesque) to DMEM (1.0 g/L Glucose) (containing L-glutamine and pyruvate) (liquid) (DMEM (LG), Nacalai Tesque), seeded in Collagen IV coated 96-well plates (Corning) at 0.1 mL per well, and cultured at 37°C in the presence of 5% by volume of CO₂.

One day after seeding and on day 4, the medium was replaced with a differentiation medium (a medium prepared by adding 0.1% by volume of FBS, 0.1% by volume of hiHS, 1% by volume of P/S, and 50 ng/mL of Mouse NGF 2.5S Native Protein (NGF, Thermo Fisher Scientific) to DMEM (LG)) to induce neuronal differentiation.

On day 5 after seeding, the medium was replaced with a differentiation medium containing 2% by volume of the test sample, and the cells were cultured at 37°C for 1 hour in the presence of 5% by volume of CO₂.

Thereafter, the medium was replaced with a differentiation medium containing 60 µM of β Amyloid 1-42 (amyloid β, Sigma-Aldrich) (neurocytotoxicity) and 2% by volume of the test sample, and the cells were cultured at 37°C for 24 hours in the presence of 5% by volume of CO₂.

After the culture, the intracellular ATP amount was measured. The measurement method was as follows. "Cell" ATP Assay reagent Ver. 2 (Toyo B-Net Co., Ltd.) was added at 0.1 mL/well, and the mixture was stirred for 1 minute with a plate shaker. The mixture was incubated at 23°C for 10 minutes, 100 µL of the mixture was transferred to a 96-well black plate, and the luminescence was measured. The cell viability was determined by dividing each luminescence by the average value of the luminescence in five tests without amyloid β treatment and multiplying the result by 100.

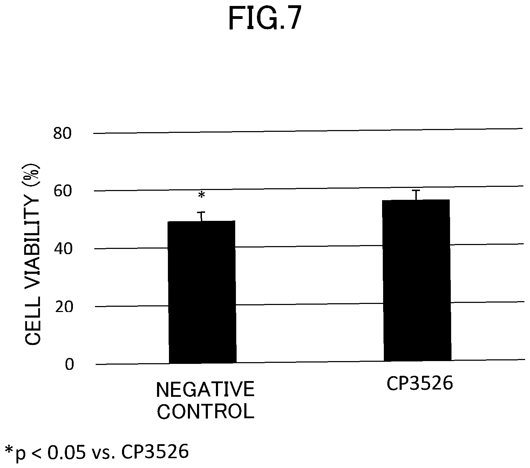

Student's t-test was used for statistical processing. The test was performed in comparison with the cell viability in the CP3526 group. A p-value<0.05 was considered statistically significant. The sample size (n) was 5. The results of cell viability are shown as the average of the values calculated in five tests. The results are shown in Table 8 and Fig. 7.

**Table 8**

| Group | Cell Viability (%) | | |
|---|---|---|---|
| | Average | Standard Deviation | p-Value |
| Negative Control | 49.05 | 3.06 | 0.021 |
| CP3526 | 55.59 | 3.39 | - |

As shown in Table 8 and Fig. 7, addition of outer membrane vesicles significantly improved the viability of neuronal cells after amyloid β treatment compared to no addition. Therefore, it was shown that the outer membrane vesicles derived from Lacticaseibacillus paracasei strain CP3526 according to the present invention protect neuronal cells from neurocytotoxicity. This suggested that outer membrane vesicles derived from specific species of lactic acid bacteria according to the present invention have useful effects on neuronal cell damage caused by neurocytotoxicity.

### [Industrial Applicability]

The present invention is applicable to pharmaceuticals, foods and/or drinks, and the like.

## Claims

1. An FPR2 agonist comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus.

2. The FPR2 agonist according to claim 1, wherein the lactic acid bacterium is at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum,
Ligilactobacillus salivarius,
Lactococcus lactis subsp. lactis,
Streptococcus salivarius subsp. thermophilus,
Lactobacillus gasseri,
Lactobacillus amylovorus,
Lactobacillus crispatus,
Lactobacillus johnsonii,
Lacticaseibacillus casei, and
Lactiplantibacillus pentosus.

3. The FPR2 agonist according to claim 1 or 2, wherein the outer membrane vesicles derived from the lactic acid bacterium do not have FPR1 agonistic activity.

4. A food and/or drink for FPR2 activation, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

5. A pharmaceutical composition for treating a disease associated with FPR2, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

6. A cosmetic composition for FPR2 activation, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

7. A consumer product composition for FPR2 activation, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

8. The food and/or drink according to claim 4, the pharmaceutical composition according to claim 5, the cosmetic composition according to claim 6, or the consumer product composition according to claim 7, wherein the outer membrane vesicles derived from the lactic acid bacterium do not have FPR1 agonistic activity.

9. Lacticaseibacillus paracasei CP3526 strain having accession number NITE BP-03625.

10. A food and/or drink for inducing IL-10 by FPR2 activation, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

11. A pharmaceutical composition for treating a disease associated with IL-10 by IL-10 induction by FPR2 activation, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

12. A cosmetic composition for inducing IL-10 by FPR2 activation, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

13. A consumer product composition for inducing IL-10 by FPR2 activation, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

14. The food and/or drink according to claim 10, the pharmaceutical composition according to claim 11, the cosmetic composition according to claim 12, or the consumer product composition according to claim 13, wherein the outer membrane vesicles derived from the lactic acid bacterium do not have FPR1 agonistic activity.

15. A food and/or drink for protecting the skin from ultraviolet rays, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

16. A pharmaceutical composition for protecting the skin from ultraviolet rays, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

17. A cosmetic composition for protecting the skin from ultraviolet rays, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

18. A consumer product composition for protecting the skin from ultraviolet rays, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

19. The food and/or drink according to claim 15, the pharmaceutical composition according to claim 16, the cosmetic composition according to claim 17, or the consumer product composition according to claim 18, which protects the skin from ultraviolet rays by FPR2 activation.

20. The food and/or drink according to claim 15, the pharmaceutical composition according to claim 16, the cosmetic composition according to claim 17, or the consumer product composition according to claim 18, wherein the outer membrane vesicles derived from the lactic acid bacterium do not have FPR1 agonistic activity.

21. A food and/or drink for protecting neuronal cells from neurocytotoxicity, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

22. A pharmaceutical composition for protecting neuronal cells from neurocytotoxicity, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

23. A cosmetic composition for protecting neuronal cells from neurocytotoxicity, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

24. A consumer product composition for protecting neuronal cells from neurocytotoxicity, comprising outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus as an active ingredient.

25. The food and/or drink according to claim 21, the pharmaceutical composition according to claim 22, the cosmetic composition according to claim 23, or the consumer product composition according to claim 24, which protects neuronal cells from neurocytotoxicity by FPR2 activation.

26. The food and/or drink according to claim 21, the pharmaceutical composition according to claim 22, the cosmetic composition according to claim 23, or the consumer product composition according to claim 24, wherein the neurocytotoxicity is neurocytotoxicity caused by amyloid β.

27. The food and/or drink according to claim 21, the pharmaceutical composition according to claim 22, the cosmetic composition according to claim 23, or the consumer product composition according to claim 24, wherein the outer membrane vesicles derived from the lactic acid bacterium do not have FPR1 agonistic activity.

28. A method of activating FPR2 in a subject, comprising
administering to the subject outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus.

29. A method of treating a disease associated with FPR2 in a subject, comprising
administering to the subject outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus.

30. A method of inducing IL-10 by FPR2 activation in a subject, comprising
administering to the subject outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus.

31. A method of protecting the skin from ultraviolet rays in a subject, comprising
administering to the subject outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus.

32. The method according to claim 31, which protects the skin from ultraviolet rays by FPR2 activation.

33. A method of protecting neuronal cells from neurocytotoxicity in a subject, comprising administering to the subject outer membrane vesicles derived from at least one lactic acid bacterium selected from the group consisting of
genus Lactobacillus,
genus Lacticaseibacillus,
genus Lactiplantibacillus,
genus Ligilactobacillus,
genus Lactococcus, and
Streptococcus salivarius subsp. thermophilus.

34. The method according to claim 33, which protects neuronal cells from neurocytotoxicity by FPR2 activation.

35. The method according to any one of claims 28 to 34, wherein the lactic acid bacterium is at least one selected from the group consisting of the following:
Lactobacillus delbrueckii subsp. bulgaricus,
Lactobacillus helveticus,
Lactobacillus acidophilus,
Lacticaseibacillus rhamnosus,
Lacticaseibacillus paracasei,
Lactiplantibacillus plantarum,
Ligilactobacillus salivarius,
Lactococcus lactis subsp. lactis,
Streptococcus salivarius subsp. thermophilus,
Lactobacillus gasseri,
Lactobacillus amylovorus,
Lactobacillus crispatus,
Lactobacillus johnsonii,
Lacticaseibacillus casei, and
Lactiplantibacillus pentosus.

36. The method according to any one of claims 28 to 35, wherein the outer membrane vesicles derived from the lactic acid bacterium do not have FPR1 agonistic activity.
